# EUROPEAN PATENT SPECIFICATION

(11) **EP 2 066 680 B1**
(45) Date of publication and mention of the grant of the patent: **08.08.2012**
(21) Application number: 07834884.4
(22) Date of filing: 26.09.2007
(51) Int. Cl.: C07F 9/28, A61K 33/42, A61P 7/02, A61P 9/10, A61P 21/02, A61P 25/00, A61P 25/28, A61P 35/00, A61P 35/04

(54) **TRIPHENYLPHOSPHONIUM THIONITRITE NITRIC OXIDE DONORS**
TRIPHENYLPHOSPHON-THIONITRIT-STICKOXID-DONATOREN
DONNEURS D'OXYDE NITRIQUE DE TYPE THIONITRITE DE TRIPHÉNYLPHOSPHONIUM

(30) Priority: 28.09.2006 US 847686 P
(43) Date of publication of application: 10.06.2009
(73) Proprietor: MEDICAL RESEARCH COUNCIL, London W1N 4AL (GB); University of Otago, Dunedin (NZ)
(72) Inventor: SMITH, Robin, Andrew, James, Dunedin (NZ); MURPHY, Michael, Patrick, Cambridge CB1 7TS (GB)
(74) Representative: Watson, Robert James
(86) International application number: PCT/NZ2007/000282
(87) International publication number: WO 2008/039085

(56) References cited:
- SEEL F. ET AL: 'The reaction of polysulfides with nitrogen monoxide in non-aqueous solvents - nitrosodisulfides' ZEITSCHRIFT FUER NATURFORSCHUNG, TEIL B: ANORGANISCHE CHEMIE, ORGANISCHE CHEMIE vol. 40B, no. 6, 1985, pages 762 - 764, XP008104950

## Description

### TECHNICAL FIELD

The invention relates to nitric oxide donor compounds and uses thereof, in particular nitric oxide donor compounds comprising a thionitrite conjugated to triphenylphosphonium cation.

### BACKGROUND OF INVENTION

The endogenous formation of nitric oxide (NO) plays a key role in many bio-regulatory systems including immune stimulation, platelet inhibition, neurotransmission, and smooth muscle relaxation (Wang, P. G., Xian, M., Tang, X., Wu, X., Wen, Z., Cai, T., Janczuk, A. J. Chem. Rev. 2002, 102, 1091-1134). Due to the instability and inconvenience of handling aqueous solutions of NO, there is increasing interest in compounds capable of generating NO *in situ,* i.e., NO donors.

All nitrogen-oxygen bonded compounds have the potential to decompose, be oxidized, or be reduced to produce reactive nitrogen species. Accordingly, a diverse range of NO donors has been developed including organic nitrates and nitrites, metal-NO complexes, N-nitrosamines, thionitrites, furoxans and benzofuroxans, oximes and N-hydroxyguanidines. However, the NO donors developed to date are poorly taken up by cells and are not targeted to particular compartments of the cell. Therefore, the presently known NO donors mainly produce NO in the circulation and thus expose a range of NO receptors to NO.

In addition to releasing free NO, NO donors can also transfer a nitrosonium group to a protein thiol to form an S-nitrosylated product. The modulation of S-nitrosylation may be involved in regulating a number of pathways important in cell metabolism (Nature Reviews:Molecular Cell Biology, 2005, 6:2, pp 150-166).

Recently, it has been shown that cytochrome c oxidase (complex IV of the mitochondrial respiratory chain) is reversibly inhibited by NO (Brown, G. C. and Cooper, C. E. FEBS Lett. 1994, 356, 295 - 298; Cleeter, M. J. W., Cooper, J. M., Darley-Usmer, V. M., Moncada, S. and Schapira, A. H. V. FEBS Lett. 1994, 345, 50-54; Schweizer, M. and Richter, C. Biochem. Biophys. Res. Commum. 1994, 204, 169-175).

This may be a physiologically relevant mechanism to limit oxygen consumption, mitochondrial ROS (reactive oxygen species) production, apoptosis and mitochondrial biogenesis and morphology.

Transfer of the nitrosonium group to protein thiols may also be involved in regulating mitochondrial function, for example at complex I (Dahm, C. C., Moore, K., Murphy, M. P., J. Biol. Chem. 2006, 281, pp 10056-10065). S-Nitrosylation of particular mitochondrial thiol proteins such as complex I may play a protective role in conditions such as ischaemia-reperfusion injury (Journal of Molecular and Cellular Cardiology, 2007, 42:4, pp 812-825).

In order to investigate this function of NO, it would be useful to have a NO donor compound that selectively released NO in the mitochondria. In addition, a number of medical conditions are influenced by the selective, reversible inhibition of mitochondria. Therefore a mitochondrially targeted NO donor would have potential as a therapeutic agent.

It is therefore an object of the invention to provide a targeted NO donor compound, or at least to provide the public with a useful choice.

### SUMMARY OF INVENTION

In one aspect the invention provides a compound of formula (I) 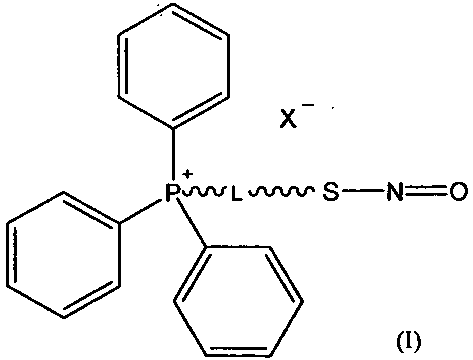 wherein -L- is a linker group and X is an optional anion. In another aspect the invention provides a compound of formula (I) 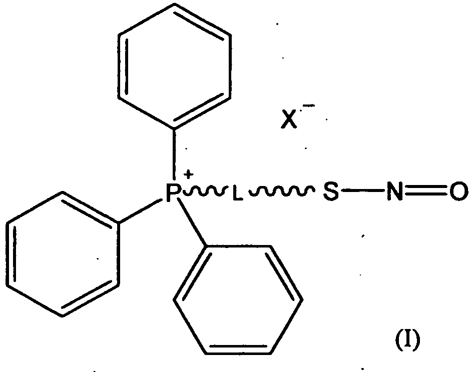 wherein -L- is a linker group and X is an optional anion where the linker group is selected from the group comprising
(a) (C₁-C₃₀) alkylene,
(b) (C₁-Cₓ) alkylene-NR-(C₁-C_{y}) alkylene, wherein R is H, alkyl, or aryl,
(c) (C₁-Cₓ) alkylene-NR-C(=O)-(C₁-C_{y}) alkylene, wherein R is H, alkyl, or aryl,
(d) (C₁-Cₓ) alkylene-C(=O)-NR-(C₁-C_{y}) alkylene, wherein R is H, alkyl, or aryl,
(e) (C₁-Cₓ) alkylene-O-(C₁-C_{y}) alkylene,
(f) (C₁-Cₓ) alkylene-O-C(=O)-(C₁-C_{y}) alkylene,
(g) (C₁-Cₓ) alkylene-S-(C₁-C_{y}) alkylene, and
(h) (C₁-Cₓ) alkylene-aryl-(C₁-C_{y}) alkylene,
wherein x + y = 30 and wherein the alkylene group is optionally substituted with one or more functional groups independently selected from the group consisting of hydrogen, alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, oxy, amino, alkylamino, aminocarbonyl, alkoxycarbonyl, aryloxycarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl, or the substituent groups of adjacent carbon atoms in the linker group can be taken together with the carbon atoms to which they are attached to form a carbocycle or a heterocycle.

In another aspect the invention provides a compound of formula (II) 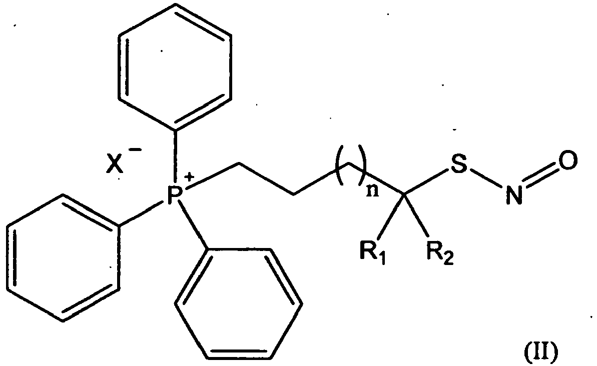 wherein n is from 0 to 27, X is an optional anion and R₁ and R₂ are independently selected from the group comprising hydrogen, alkyl and aryl.

In another aspect the invention provides a compound of formula (III) 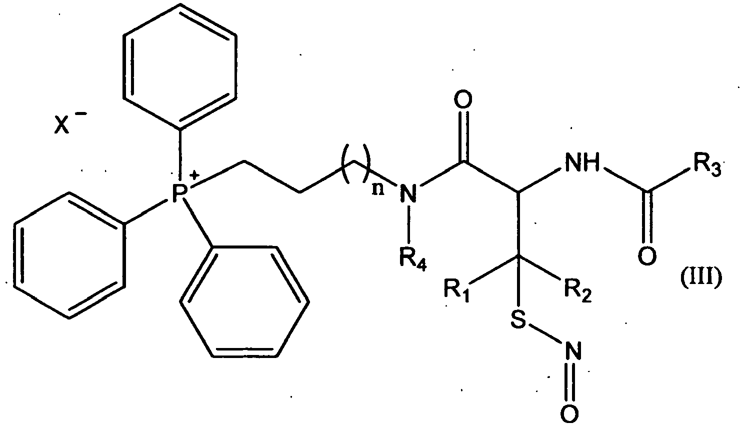 wherein n is from 0 to 27, X is an optional anion and R₁, R₂, R₃ and R₄ are independently selected from the group comprising hydrogen, alkyl and aryl.

In another aspect the invention provides a compound of formula (IV) 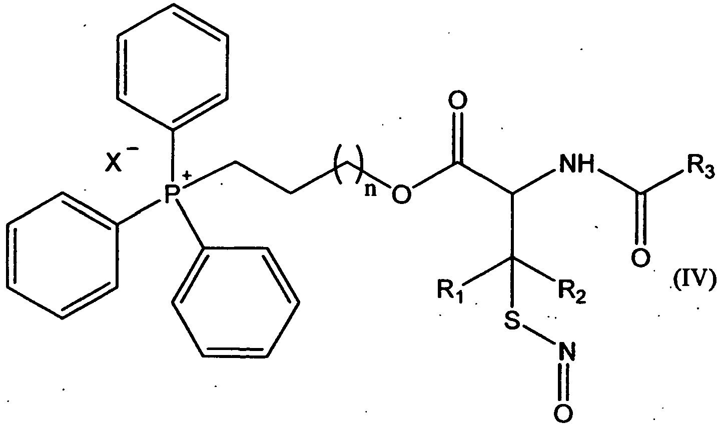 wherein n is from 0 to 27, X is an optional anion and R₁, R₂ and R₃ are independently selected from the group comprising hydrogen, alkyl and aryl.

In another aspect the invention provides a compound of formula (V) 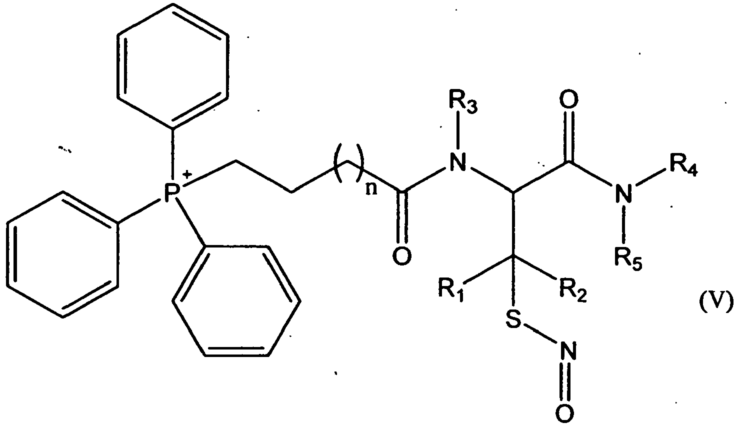 wherein n is from 0 to 24, X is an optional anion and R₁, R₂, R₃, R₄ and R₅ are independently selected from the group comprising hydrogen, alkyl and aryl.

In another aspect the invention provides a compound of formula (VI) 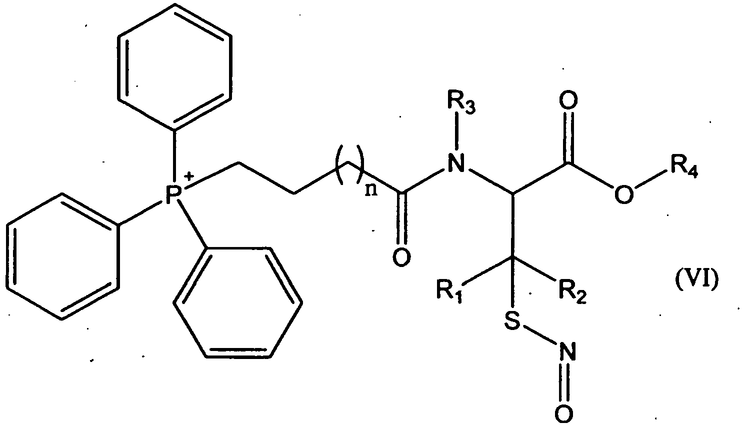 wherein n is from 0 to 24, X is an optional anion and R₁, R₂, R₃, and R₄ are independently selected from the group comprising hydrogen, alkyl and aryl.

In another aspect the invention provides a pharmaceutical composition comprising a therapeutically effective amount of a compound of the invention in combination with one or more pharmaceutically acceptable excipients, carriers or diluents.

In another aspect the invention provides a use of a compound of the invention in the preparation of a medicament for the treatment of ischaemia-reperfusion injury.

In another aspect the invention provides a use of a compound of the invention in the preparation of a medicament for inhibiting angiogenesis.

In another aspect the invention provides a use of a compound of the invention in the preparation of a medicament for treating neoplasms, tumour growth and/or metastasis.

In another aspect the invention provides a use of a compound of the invention in the preparation of a medicament for the treatment of cancer.

In another aspect the invention provides a use of a compound of the invention in the preparation of a medicament for the treatment of Alzheimer's disease.

In another aspect the invention provides a use of a compound of the invention in the preparation of a medicament for the treatment of Parkinson's disease.

In another aspect the invention provides a use of a compound of the invention in the preparation of a medicament for generating NO in the mitochondria of a subject.

In another aspect the invention provides a use of a compound of the invention in the preparation of a medicament for S-nitrosylating proteins in the mitochondria of a subject.

In another aspect the invention provides a use of a compound of the invention in the preparation of a medicament for inhibiting cytochrome oxidase in the mitochondria of a subject.

In another aspect the invention provides a method of treating ischaemia-reperfusion injury in a subject in need thereof, said method comprising administering to the subject a therapeutically effective amount of a compound of the invention or a pharmaceutical composition comprising same.

In another aspect the invention provides a method of inhibiting angiogenesis in a subject in need thereof, said method comprising administering to the subject a therapeutically effective amount of a compound of the invention or a pharmaceutical composition comprising same.

In another aspect the invention provides a method of treating neoplasms, tumour growth and/or metastasis in a subject in need thereof, said method comprising administering to the subject a therapeutically effective amount of a compound of the invention or a pharmaceutical composition comprising same.

In another aspect the invention provides a method of treating cancer in a subject in need thereof, said method comprising administering to the subject a therapeutically effective amount of a compound of the invention or a pharmaceutical composition comprising same.

In another aspect the invention provides a method of treating Alzheimer's disease in a subject in need thereof, said method comprising administering to the subject a therapeutically effective amount of a compound of the invention or a pharmaceutical composition comprising same.

In another aspect the invention provides a method of treating Parkinson's disease in a subject in need thereof, said method comprising administering to the subject a therapeutically effective amount of a compound of the invention or a pharmaceutical composition comprising same.

In another aspect the invention provides a method for generating NO in the mitochondria of a subject comprising administering to the subject, a therapeutically effective amount of a compound of the invention or a pharmaceutical composition comprising same.

In another aspect the invention provides a method for S-nitrosylating proteins in the mitochondria of a subject comprising administering to the subject, a therapeutically effective amount of a compound of the invention or a pharmaceutical composition comprising same.

In another aspect the invention provides a method for inhibiting cytochrome oxidase in the mitochondria of a subject comprising administering to the subject, a therapeutically effective amount of a compound of the invention or a pharmaceutical composition comprising same.

### BRIEF DESCRIPTION OF THE DRAWINGS

Preferred features of the invention will be described, by way of example only, with reference to the following figures.
**Figure 1** is a schematic diagram showing the effects of a compound of the invention (MitoSNO) when delivered to a cell. MitoSNO accumulates within the mitochondria and releases NO, inhibiting cytochrome oxidase by competing with oxygen.
**Figure 2** is a trace showing NO release from a compound of the invention (MitoSNO) measured using a NO electrode in the presence and absence of glutathione (GSH). The trace shown is typical of two experiments.
**Figure 3** is a trace showing MitoSNO uptake into energized respiring mitochondria in a Δψ-dependent manner. Oxygen (black trace) and TPP cation (dashed trace) concentrations were measured simultaneously. The trace shown is typical of two experiments.
**Figure 4** is a trace showing MitoSNO uptake into respiring mitochondria in a Δψ-dependent manner. Oxygen (black trace) and TPP (triphenylphosphonium) cation (dashed trace) concentrations were measured. The trace shown is typical of two experiments. MitoSNO uptake leads to production of NO inside the mitochondria which gradually inhibits respiration, as indicated by the shape of the oxygen electrode trace. This also decreases the membrane potential, as shown by the decreased uptake of MitoSNO.
**Figure 5** is a graph showing the concentration of nitrate formed over time from a compound of the invention (MitoSNAP) compared to SNAP. The NO released from MitoSNAP reacts with oxygen to form nitrite.

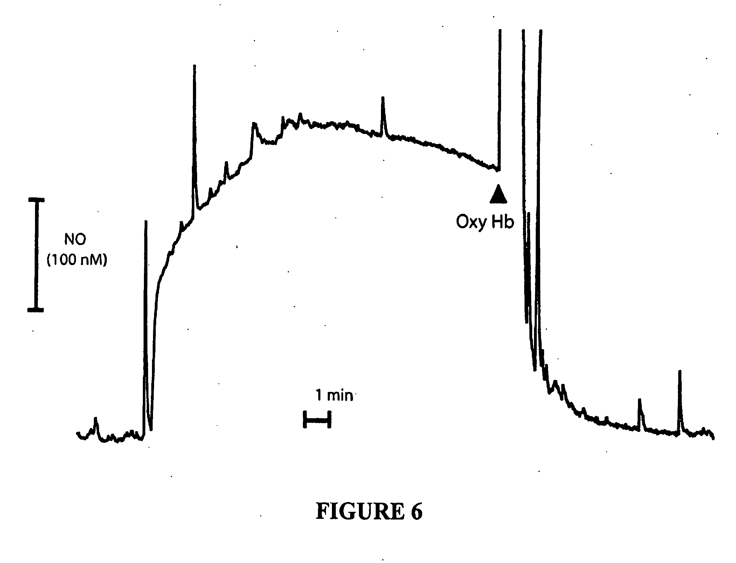

**Figure 6** is a trace showing release of NO from MitoSNAP, as assessed by measuring the formation of NO with an NO electrode then degrading the NO to nitrate by addition of oxyhemoglobin (Oxy Hb).

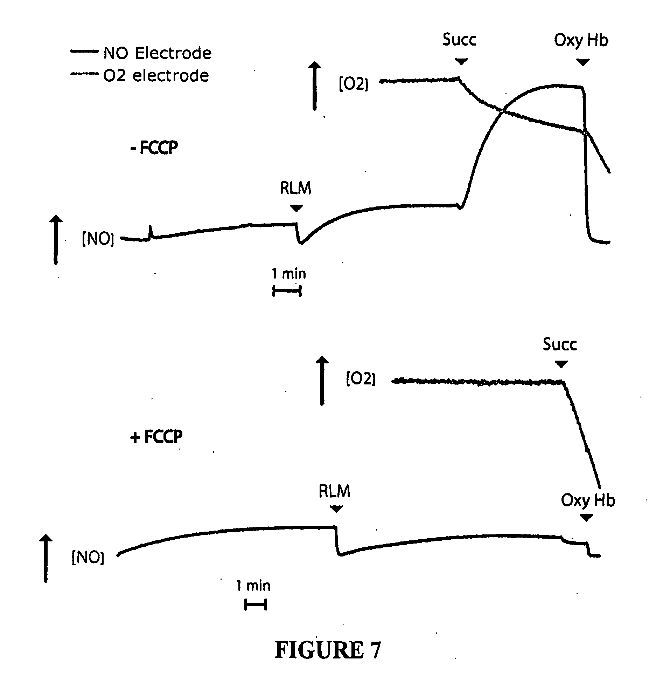

**Figure 7** is a trace showing the interation of isolated mitochondria with MitoSNAP. The trace shows the results from an NO-electrode (measuring NO) and an oxygen electrode (measuring respiration of the mitochondria). In the upper trace; addition of succinate to energise the mitochondria leads to a large increase in NO formation due to accumulation of MitoSNAP into the mitochondria and subsequent NO release. The released NO then interacts with cytochrome oxidase to decrease respiration, as indicated by the curving off of the oxygen electrode trace. In contract, in the lower trace, the effects of MitoSNAP on NO production and inhibition of respiration are much lower in the presence of uncoupling molecules FCCP. FCCP abolishes the membrane potential preventing uptake of MitoSNAP into the mitochondria.

### DETAILED DESCRIPTION OF THE INVENTION

### 1. Definitions

The term "alkyl," by itself or as part of another substituent, means, unless otherwise stated, a straight or branched chain, or cyclic hydrocarbon radical, or combination thereof, which may be fully saturated, mono- or polyunsaturated and can include di- and multivalent radicals, having the number of carbon atoms designated (i.e. C₁-C₁₀ means one to ten carbons). Examples of saturated hydrocarbon radicals include groups such as methyl, ethyl, n-propyl, isopropyl, n-butyl, t-butyl, isobutyl, sec-butyl, cyclohexyl, (cyclohexyl)ethyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below as "cycloalkyl" and "alkylene." The term "alkylene" by itself or as part of another substituent means a divalent radical derived from an alkane, as exemplified by -CH₂CH₂CH₂CH₂-. Typically, an alkyl group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The terms "cycloalkyl" by itself or in combination with other terms, represent, unless otherwise stated, a cyclic versions of "alkyl". Examples of cycloalkyl include cyclopentyl, cyclohexyl, 1-cyclohexenyl, 3-cyclohexenyl, cycloheptyl, and the like.

The term "alkenyl", as used herein, means a hydrocarbon radical having at least one double bond including, but not limited to, ethenyl, propenyl, 1-butenyl, 2-butenyl and the like.

The term "alkynyl", as used herein, means a hydrocarbon radical having at least one triple bond including, but not limited to, ethynyl, propynyl, 1-butynyl, 2-butynyl and the like.

The terms "halo" or "halogen," by themselves or as part of another substituent, mean, unless otherwise stated, a fluorine, chlorine, bromine, or iodine atom. Additionally, terms such as "fluoroalkyl," are meant to include monofluoroalkyl and polyfluoroalkyl.

The term "aryl," employed alone or in combination with other terms (e.g., aryloxy, arylthioxy, arylalkyl) means, unless otherwise stated, an aromatic substituent which can be a single ring or multiple rings (up to three rings) which are fused together or linked covalently. The rings may each contain from zero to four heteroatoms selected from N, O, and S, wherein the nitrogen and sulfur atoms are optionally oxidized, and the nitrogen atom(s) are optionally quaternized. The aryl groups that contain heteroatoms may be referred to as "heteroaryl" and can be attached to the remainder of the molecule through a heteroatom. Non-limiting examples of aryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl 4-pyrimidyl, 2-benzothiazolyl, 5-benzothiazolyl, 2-benzoxazolyl, 5-benzoxazolyl, purinyl, 2-benzimidazolyl, 5-indolyl, 1-isoquinolinyl, 5-isoquinolinyl, 2-quinoxalinyl, 5-quinoxalinyl, 3-quinolinyl, and 6-quinolinyl. Substituents for each of the above noted aryl ring systems are selected from the group of acceptable substituents described herein.

The term "alkoxy", as used herein, means an O-alkyl group wherein "alkyl" is defined above.

The term "sulfonyl" refers to a radical -S(O)₂R where R is an alkyl, substituted alkyl, substituted cycloalkyl, substituted heterocycloalkyl, substituted aryl, or substituted heteroaryl group as defined herein. Representative examples include, but are not limited to methylsulfonyl, ethylsulfonyl, propylsulfonyl, butylsulfonyl, and the like.

The term "sulfinyl" refers to a radical -S(O)R where R is an alkyl, substituted alkyl, substituted cycloalkyl, substituted heterocycloalkyl, substituted aryl, or substituted heteroaryl group as defined herein. Representative examples include, but are not limited to, methylsulfinyl, ethylsulfinyl, propylsulfinyl, butylsulfinyl, and the like.

The term "aralkyl" or "arylalkyl" means an aryl-alkyl- group in which the aryl and alkyl are as previously described. Preferred aralkyls comprise a lower alkyl group attached to the aryl group. Non-limiting examples of suitable aralkyl groups include phenymethylene, 2-phenethyl and naphthalenylmethyl. The bond to the parent moiety is through the alkyl.

The term "pharmaceutically acceptable" as used herein refers to compounds, ingredients, materials, compositions, dosage forms and the like, which are within the scope of sound medical judgment, suitable for use in contact with the tissues of the subject in question (e.g. human) without excessive toxicity, irritation, allergic response, or other problem or complication, commensurate with a reasonable benefit/risk ratio. Each carrier, diluent, exipient, etc., must also be "acceptable" in the sense of being compatible with the other ingredients of the formulation.

The term "subject" as used herein refers to a human or non-human mammal. Examples of non-human mammals include livestock animals such as sheep, horses, cows, pigs, goats, rabbits, deer, ostriches and emus; and companion animals such as cats, dogs, rodents, and horses.

The term "treatment" as used herein in the context of treating a condition, pertains generally to treatment and therapy, whether of human or animal, in which some desired therapeutic effect is achieved, for example, the inhibition of progress of the condition, and includes a reduction in the rate of progress, a halt in the rate of progress, amelioration of the condition, and cure of the condition. Treatment as a prophylactic measure (i.e., prophylaxis) is also included.

"Treatment" also includes combination treatments and therapies, in which two or more treatments or therapies are combined, for example, sequentially or simultaneously. For example, a therapeutically effective amount of a compound of the invention could be combined with or used in conjunction with radiation therapy or chemotherapy in the treatment of cancer.

The term "therapeutically-effective amount" as used herein, pertains to that amount of an active compound, or a material, composition or dosage form comprising an active compound, which is effective for producing some desired therapeutic or prophylactic effect, commensurate with a reasonable benefit/risk ratio.

As used herein the term "comprising" means "consisting at least in part of". When interpreting each statement in this specification that includes the term "comprising", features other than that or those prefaced by the term may also be present. Related terms such as "comprise" and "comprises" are to be interpreted in the same manner.

The statements wherein, for example, R¹, R² and R³, are said to be independently selected from a group of substituents, mean that R^{1,} R² and R³ are independently selected, but also that where an R, R¹, R² and R³ variable occurs more than once in a molecule, each occurrence is independently selected (e.g., if R is -OR⁶ wherein R⁶ is hydrogen, R² can be -OR⁶ wherein R⁶ is lower alkyl). Those skilled in the art will recognize that the size and nature of the substituent(s) will affect the number of substituents that can be present.

Some compounds of the invention have at least one asymmetrical carbon atom and therefore all isomers, including enantiomers, stereoisomers, rotamers, tautomers and racemates of the compounds are contemplated as being part of this invention. The invention includes d and 1 isomers in both pure form and in admixture, including racemic mixtures. Isomers can be prepared using conventional techniques, either by reacting optically pure or optically enriched starting materials or by separating isomers of a compound of the invention. Isomers may also include geometric isomers, e.g., when a double bond is present.

Those skilled in the art will appreciate that for some of the compounds of the invention, one isomer may show greater pharmacological activity than other isomers.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### 2. Compounds of the invention

Thionitrites (RSNO) are an important class of NO donor molecules. They are produced in biological systems in response to NO and are believed to play an important role in storing, transporting and releasing NO under physiological conditions. It has now been shown that conjugation of a thionitrite-containing molecule to a triphenylphosphonium cation produces a compound that rapidly enters the cell through direct passage through the plasma membrane.

These compounds enable production of NO in the thiol reducing environment within the cell. Therefore, use of the triphenylphosphonium cation compounds of the invention may enable more efficient and regulated intracellular production of NO. Intracellular production of NO may have applications for regulating signaling pathways dependent on NO such as vasodilation, regulating activity of intracellular signaling pathways that impact on cell function and on the regulation of mitochondrial biogenesis.

In addition, NO itself may act as an antioxidant, therefore the intracellular production of NO may to protect against oxidative damage in pathologies (Current Medicinal Chemistry: Anti-inflammatory and Anti-Allergy agents, 2004, 3:3, pp 181-188).

The compounds of the invention not only release free NO but can also transfer a nitrosonium group to a protein thiol, thereby forming a S-nitrosylated product. Therefore, the compounds of the invention allow for manipulation of both free NO and the S-nitrosylation status of cells, in order to manipulate and modify the cell's metabolism and function.

In addition to rapidly causing NO release inside the cell, conjugation to a lipophilic cation enables the NO donor to accumulate within the mitochondria. Therefore, the compounds of the invention can selectively release NO in the mitochondria and/or can S-nitrosylate proteins in the mitochondria.

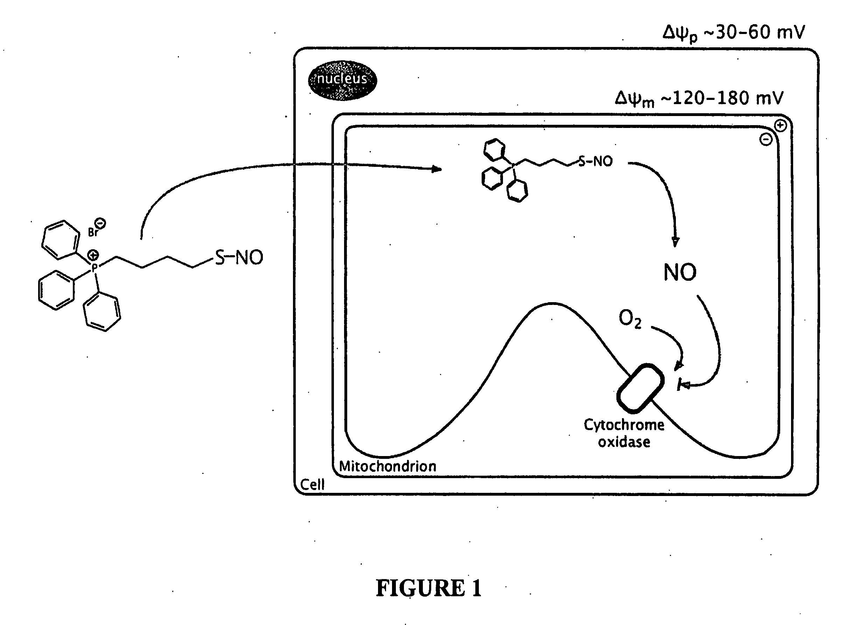

Mitochondria have a substantial membrane potential of up to 180 mV across their inner membrane (negative inside). Because of the potential, membrane permeant lipophilic cations such as the triphenylphosphonium cation accumulate several-hundred fold within the mitochondrial matix. This is illustrated in Figure 1 which shows the effect of a compound of the invention (MitoSNO) when delivered to a whole cell.

MitoSNO is taken up by cells, courtesy of the plasma membrane potential (Δψp), and further accumulates in the mitochondrial matrix, driven by the mitochondrial membrane potential (Δψm). Once inside mitochondria, MitoSNO releases NO, which results in direct inhibition of respiration at complex (IV) of the respiratory chain (Brown, G. C. and Cooper, C. E. FEBS Lett. 1994, 356, 295 - 298; Cleeter, M. J. W., Cooper, J. M., Darley-Usmer, V. M., Moncada, S. and Schapira, A. H. V. FEBS Lett. 1994, 345, 50-54; Schweizer, M. and Richter, C. Biochem. Biophys. Res. Commum. 1994, 204, 169-175).

Previously, NO donors have been used to modify blood pressure. However, these NO donors mainly produce NO in the circulation and thus expose a range of NO receptors to NO, thereby affecting blood pressure as well as mitochondria. The compounds of the present invention selectively produce NO within the mitochondria, thereby focusing on the NO effects on the respiratory chain.

In one aspect the invention provides a compound of formula (I) 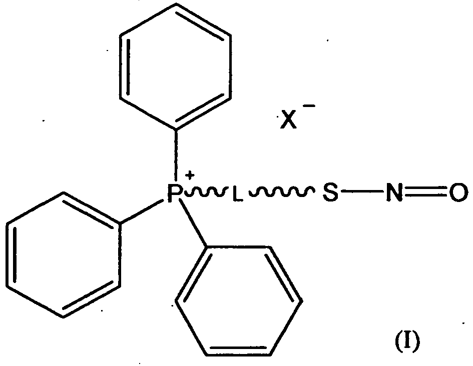 wherein -L- is a linker group and X is an optional anion.

The linker group ~L~ may be any chemically non-active distance-making group (spacer) which joins the triphenylphosphonium cation moiety to the thionitrite moiety, and enables the two moieties to remain bonded together when crossing the plasma and mitochondrial membranes. In particular ~L~ is stable under physiological conditions.

In one embodiment the linker group will be an alkylene group. The term "alkylene" as used herein, means a bidentate moiety obtained by removing two hydrogen atoms, either both from the same carbon atom, or one from each of two different carbon atoms, of a hydrocarbon compound having from 1 to 30 carbon atoms, preferably 2 to 20, more preferably 2 to 10, even more preferably 3 to 5 and most preferably 4, which may be aliphatic or alicyclic, and which may be saturated, partially unsaturated, or fully unsaturated. Thus, the term "alkylene" includes the sub-classes alkenylene, alkynylene, and cycloalkylene.

In one embodiment the linker group is (C₁-C₃₀) alkylene or substituted (C₁-C₃₀) alkylene. Preferably the linker group is (C₂-C₂₀) alkylene or substituted (C₂-C₂₀) alkylene. More preferably, the linker group is (C₂-C₁₀) alkylene or substituted (C₂-C₁₀) alkylene. Most preferably, the linker group is (C₃-C₅) alkylene or substituted (C₃-C₅) alkylene.

The linker group may also be substituted by one or more substituent groups that increases the solubility of the molecule, increases the uptake of the molecule across the plasma and/or mitochondrial membranes, or decreases the rate of degradation of the molecule *in vivo.*

In one embodiment the linker group is substituted with one or more functional groups independently selected from the group consisting of hydrogen, alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, oxy, amino, alkylamino, aminocarbonyl, alkoxycarbonyl, aryloxycarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl, or the substituent groups of adjacent carbon atoms in the linker group can be taken together with the carbon atoms to which they are attached to form a carbocycle or a heterocycle.

In one embodiment the linker group may be substituted by alkyl, hydroxyl, thio, amino, carboxy, amido groups or groups derived from sugars or sugar derivatives.

In one embodiment the linker group is alkyl or aryl substituted. Preferably the linker group is substituted with one or more substituents selected from the group comprising hydrogen, methyl, ethyl, propyl, butyl, pentyl, hexyl and aryl.

In one embodiment the linker group is substituted at the carbon α to the S atom. Preferably, the linker group is disubstituted with alkyl or aryl at the carbon α to the S atom. More preferably, the linker group is disubstituted with methyl groups at the carbon α to the S atom.

In one embodiment the linker group is substituted at the carbon β to the S atom. Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom.

In one embodiment the linker group is alkyl-substituted (C₃-C₅) alkylene, preferably alkyl substituted butylene, more preferably, dimethyl substituted butylene.

In one embodiment the linker group is (C₁-C₃₀) alkylene substituted at the carbon α to the S atom. Preferably the linker group is (C₂-C₂₀) alkylene substituted at the carbon α to the S atom. More preferably the linker group is (C₂-C₁₀) alkylene substituted at the carbon α to the S atom. Most preferably the linker group is (C₃-C₅) alkylene substituted at the carbon α to the S atom. Preferably, the linker group is dimethyl substituted at the carbon a to the S atom.

The linker group may also include within its structure, one or more aryl groups. The aryl group(s) may be positioned anywhere in the linker.

In one embodiment the linker group is an aryl-containing alkylene chain. Preferably, the linker group is (C₁-Cₓ) alkylene-aryl-(C₁-C_{y}) alkylene, or substituted (C₁-Cₓ) alkylene-aryl-substituted (C₁-C_{y}) alkylene where x + y = 30. Preferably the linker group is (C₂-C₁₅) alkylene-aryl-(C₂-C₁₅) alkylene or substituted (C₂-C₁₅) alkylene-aryl-substituted (C₂-C₁₅) alkylene. More preferably the linker group is (C₂-C₁₀) alkylene-aryl-(C₂-C₁₀) alkylene or substituted (C₂-C₁₀) alkylene-aryl-substituted (C₂-C₁₀) alkylene. Most preferably the linker group is (C₂-C₅) alkylene-aryl-(C₂-C₅) alkylene or substituted (C₂-C₅) alkylene-aryl-substituted (C₂-C₅) alkylene.

In one embodiment the aryl group of the linker is substituted. Preferably, the aryl group is substituted with one or more functional groups independently selected from the group consisting of hydrogen, alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, amino, alkylamino, aminocarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl.

The linker group may also include within its structure, one or more heteroatoms such as N, O and S. The heteroatom may be positioned anywhere in the linker.

In one embodiment the linker group is (C₁-Cₓ) alkylene-NR-(C₁-C_{y}) alkylene, or substituted (C₁-Cₓ) alkylene-NR-substituted (C₁-C_{y}) alkylene, wherein R is hydrogen, alkyl or aryl, and x + y = 30. Preferably the linker group is (C₂-C₁₅) alkylene-NR-(C₂-C₁₅) alkylene or substituted (C₂-C₁₅) alkylene-NR-substituted (C₂-C₁₅) alkylene. More preferably the linker group is (C₂-C₁₀) alkylene-NR-(C₂-C₁₀) alkylene or substituted (C₂-C₁₀) alkylene-NR-substituted (C₂-C₁₀) alkylene. Most preferably the linker group is (C₂-C₅) alkylene-NR-(C₂-C₅) alkylene or substituted (C₂-C₅) alkylene-NR-substituted (C₂-C₅) alkylene. Preferably, R is hydrogen.

Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom. Preferably, the linker group is substituted with an alkyl or aryl group at the carbon α to the S atom.

In one embodiment the linker group is (C₁-Cₓ) alkylene-NR-C(=O)-(C₁-C_{y}) alkylene, or substituted (C₁-Cₓ) alkylene-NR-C(=O)-substituted (C₁-C_{y}) alkylene, wherein R is hydrogen, alkyl or aryl and x + y = 30. Preferably the linker group is (C₂-C₁₅) alkylene-NR-C(=O)-(C₂-C₁₅) alkylene or substituted (C₂-C₁₅) alkylene-NR-C(=O)-substituted (C₂-C₁₅) alkylene. More preferably the linker group is (C₂-C₁₀) alkylene-NR-C(=O)-(C₂-C₁₀) alkylene or substituted (C₂-C₁₀) alkylene-NR-C(=O)-substituted (C₂-C₁₀) alkylene. Most preferably the linker group is (C₂-C₅) alkylene-NR-C(=O)-(C₂-C₅) alkylene or substituted (C₂-C₅) alkylene-NR-C(=O)-substituted (C₂-C_{S}) alkylene. Preferably, R is hydrogen.

Where the linker group is (C₁-Cₓ)alkylene-NR-C(=O)-(C₁-C_{y})alkylene, either end of the linker may be attached to the triphenylphosphonium ion. In other words, in one embodiment the linker group is (C₁-Cₓ) alkylene-C(=O)-NR-(C₁-C_{y}) alkylene, or substituted (C₁-Cₓ) alkylene-C(=O)NR-(C₁-C_{y}) substituted alkylene wherein R is hydrogen, alkyl or aryl and x + y = 30. Preferably the linker group is (C₂-C₁₅) alkylene-C(=O)-NR-(C₂-C₁₅) alkylene or substituted (C₂-C₁₅) alkylene-C(=O)-NR-substituted (C₂-C₁₅) alkylene. More preferably the linker group is (C₂-C₁₀) alkylene-C(=O)-NR-(C₂-C₁₀) alkylene or substituted (C₂-C₁₀) alkylene-C(=O)-NR-substituted (C₂-C₁₀) alkylene. Most preferably the linker group is (C₂-C₅) alkylene-C(=O)-NR-(C₂-C₅) alkylene or substituted (C₂-C₅) alkylene-C(=O)-NR-substituted (C₂-C₅) alkylene. Preferably, R is hydrogen.

Preferably, the linker group is orientated so that the NR is on the triphenylphosphonium end of the -C(=O)NR- group in the chain.

Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom. Preferably, the linker group is substituted with an alkyl or aryl group at the carbon α to the S atom.

In one embodiment the linker group is (C₁-Cₓ) alkylene-O-(C₁-C_{y}) alkylene, or substituted (C₁-Cₓ) alkylene-O-substituted (C₁-C_{y}) alkylene where x + y = 30. Preferably the linker group is (C₂-C₁₅) alkylene-O-(C₂-C₁₅) alkylene or substituted (C₂-C₁₅) alkylene-O-substituted (C₂-C₁₅) alkylene. More preferably the linker group is (C₂-C₁₀) alkylene-O-(C₂-C₁₀) alkylene or substituted (C₂-C₁₀) alkylene-O-substituted (C₂-C₁₀) alkylene. Most preferably the linker group is (C₂-C₅) alkylene-O-(C₂-C₅) alkylene or substituted (C₂-C₅) alkylene-O-substituted (C₂-C₅) alkylene.

Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom. Preferably, the linker group is substituted with an alkyl or aryl group at the carbon α to the S atom.

In one embodiment the linker group is (C₁-Cₓ) alkylene-O-C(O)-(C₁-C_{y}) alkylene, or substituted (C₁-Cₓ) alkylene-O-C(O)-substituted (C₁-C_{y}) alkylene where x+y=30. Preferably the linker group is (C₂-C₁₅) alkylene-O-C(O)-(C₂-C₁₅) alkylene or substituted (C₂-C₁₅) alkylene-O-C(O)-substi.tuted (C₂-C₁₅) alkylene. More preferably the linker group is (C₂-C₁₀) alkylene-O-C(O)-(C₂-C₁₀) alkylene or substituted (C₂-C₁₀) alkylene-O-C(O)-substituted (C₂-C₁₀) alkylene. Most preferably the linker group is (C₂-C₅) alkylene-O-C(O)-(C₂-C₅) alkylene or substituted (C₂-C₅) alkylene-O-C(O)-substituted (C₂-C₅) alkylene.

Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom. Preferably, the linker group is substituted with an alkyl or aryl group at the carbon α to the S atom.

In one embodiment the linker group is (C₁-Cₓ) alkylene-S-(C₁-C_{y}) alkylene, or substituted (C₁-Cₓ) alkylene-S-substituted (C₁-C_{y}) alkylene wherein x +y = 30. Preferably the linker group is (C₂-C₁₅) alkylene-S-(C₂-C₁₅) alkylene or substituted (C₂-C₁₅) alkylene-S-substituted (C₂-C₁₅) alkylene. More preferably the linker group is (C₂-C₁₀) alkylene-S-(C₂-C₁₀) alkylene or substituted (C₂-C₁₀) alkylene-S-substituted (C₂-C₁₀) alkylene. Most preferably the linker group is (C₂-C₅) alkylene-S-(C₂-C₅) alkylene or substituted (C₂-C₅) alkylene-S-substituted (C₂-C₅) alkylene.

Preferably, the linker group is substituted with a group selected from alkylcarbonylamino, arylcarbonylamino, alkylaminocarbonyl, arylaminocarbonyl, alkoxycarbonyl and aryloxycarbonyl. More preferably, the linker group is substituted with alkylcarbonylamino at the carbon β to the S atom. Preferably, the linker group is substituted with an alkyl or aryl group at the carbon α to the S atom.

Linker groups containing aryl and/or heteroatoms may also be substituted at other positions within the linker. In one embodiment the linker is optionally substituted with one or more functional groups independently selected from the group consisting of hydrogen, alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, oxy, amino, alkylamino, aminocarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl.

Preferably, the linker group is optionally substituted with one or more functional groups independently selected from the group consisting of alkyl, aryl, alkoxycarbonyl, aryloxycarbonyl, alkylaminocarbonyl, arylaminocarbonyl, alkylcarbonylamino and arylcarbonylamino.

The optional anion comprises a suitable inorganic or organic anion known in the art and is present when required for overall electrical neutrality. Examples of suitable inorganic anions include, but are not limited to, those derived from hydrochloric, hydrobromic, hydroiodic, sulfuric, sulfurous, nitric, nitrous, phosphoric or phosphorous acid or from an alkylsulfonic or an arylsulfonic acid.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyuvic, salicylic, stearic, succinic, sulfanilic, tartaric, toluenesulfonic, and valeric. All are generally recognized as pharmaceutically acceptable salts.

In one embodiment the anion is an anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide, most preferably bromide.

In another embodiment the anion is methanesulfonate.

In another aspect the invention provides a compound of formula (II) 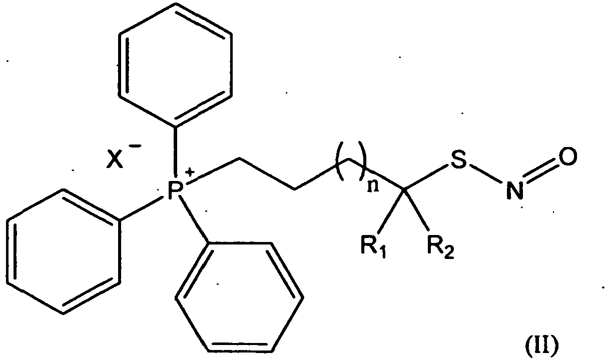 wherein n is from 0 to 27, X is an optional anion and R₁ and R₂ are independently selected from the group comprising hydrogen, alkyl and aryl.

In one embodiment n is from 0 to 20. Preferably n is from 0 to 10. More preferably n is from 0 to 2. Most preferably, n is 1.

In one embodiment R₁ and R₂ are independently selected from the group comprising hydrogen, methyl, ethyl, propyl, butyl, pentyl, hexyl and aryl. Preferably, R₁ and R₂ are methyl.

In one embodiment the anion is an inorganic anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide or methanesulfonate. Preferably, the anion is bromide or methanesulfonate.

In the compound of formula (II) the linker group comprises (C₃-C₃₀) alkylene optionally substituted with alkyl or aryl at the carbon α to the sulfur atom.

In another aspect the invention provides a compound of formula (III) 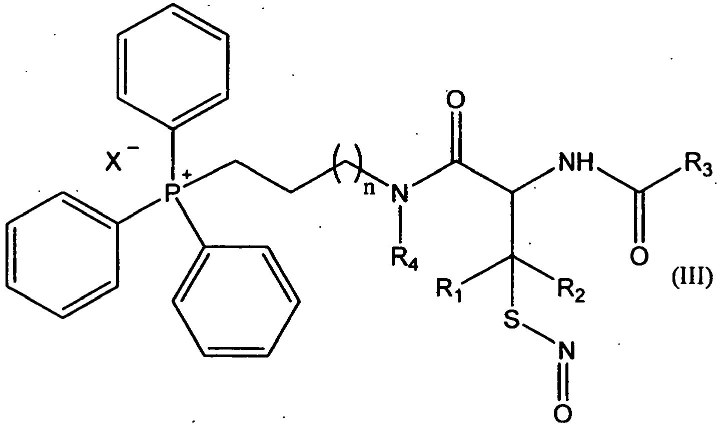 wherein n is from 0 to 27, X is an optional anion and R₁, R₂, R₃ and R₄ are independently selected from the group comprising hydrogen, alkyl and aryl.

In one embodiment n is from 0 to 20. Preferably n is from 0 to 10. More preferably n is from 1 to 5. Most preferably, n is 3.

In one embodiment R₁ R₂, R₃ and R₄ are independently selected from the group comprising hydrogen, methyl, ethyl, propyl, butyl, pentyl, hexyl and aryl. Preferably, R₁ R₂ and R₃ are methyl, and R₄ is hydrogen.

In one embodiment the anion is an inorganic anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide or methanesulfonate. Preferably, the anion is bromide or methanesulfonate.

In the compounds of formula (III) the linker group comprises (C₂-C₂₉) alkylene-NR-C(=O)-C₂ alkylene substituted with alkylcarbonylamino or arylcarbonylamino at the carbon β to the S atom and substituted with alkyl or aryl at the carbon α to the S atom.

In another aspect the invention provides a compound of formula (IV) 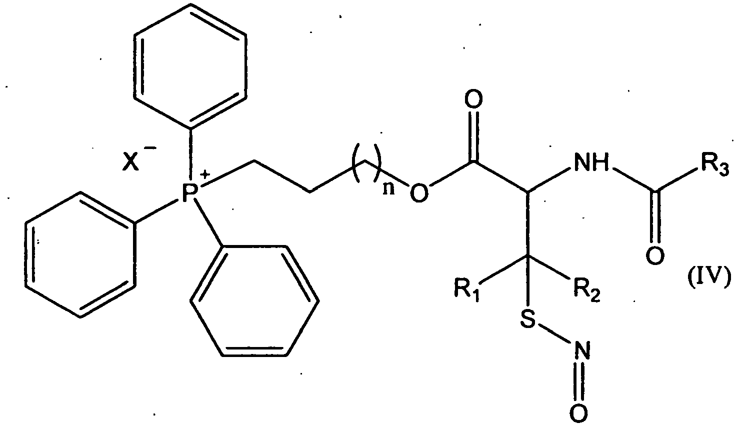 wherein n is from 0 to 27, X is an optional anion and R₁, R₂ and R₃ are independently selected from the group comprising hydrogen, alkyl and aryl.

In one embodiment n is from 0 to 20. Preferably n is from 0 to 10. More preferably n is from 1 to 5. Most preferably, n is 3.

In one embodiment R₁ R₂ and R₃ are independently selected from the group comprising hydrogen, methyl, ethyl, propyl, butyl, pentyl, hexyl and aryl. Preferably, R₁ R₂ and R₃ are methyl.

In one embodiment the anion is an inorganic anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide or methanesulfonate. Preferably, the anion is bromide or methanesulfonate.

In the compound of formula (IV) the linker group comprises (C₂-C₂₉) alkylene-O-C(=O)-C₂ alkylene substituted with alkylcarbonylamino at the carbon β to the S atom and substituted with alkyl or aryl at the carbon α to the S atom.

In another aspect the invention provides a compound of formula (V) 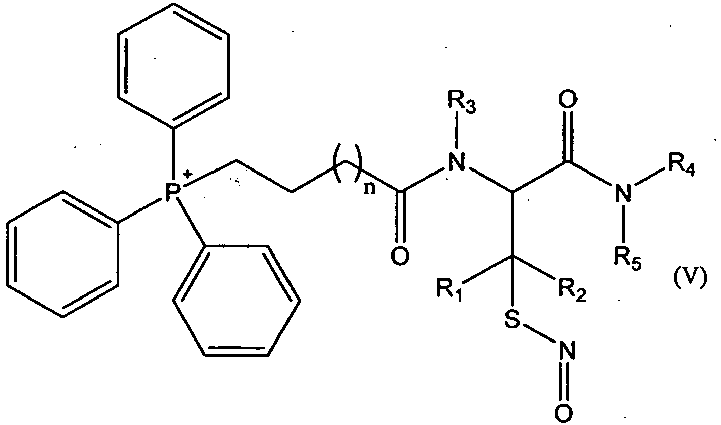 wherein n is from 0 to 27, X is an optional anion and R₁, R₂, R₃, R₄ and R₅ are independently selected from the group comprising hydrogen, alkyl and aryl.

In one embodiment n is from 0 to 20. Preferably n is from 0 to 10. More preferably n is from 1 to 5. Most preferably, n is 3.

In one embodiment R₁ R₂, R₃, R₄ and R₅ are independently selected from the group comprising hydrogen, methyl, ethyl, propyl, butyl, pentyl, hexyl and aryl. Preferably, R₁ R₂, R₃, R₄ and R₅ are methyl.

In one embodiment the anion is an inorganic anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide or methanesulfonate. Preferably, the anion is bromide or methanesulfonate.

In the compound of formula (V) the linker group comprises (C₂-C₂₉) alkylene-C(=O)-NR-C₂ alkylene substituted with alkylaminocarbonyl or arylcarbonylamino at the carbon β to the S atom and substituted with alkyl or aryl at the carbon α to the S atom.

In another aspect the invention provides a compound of formula (VI) 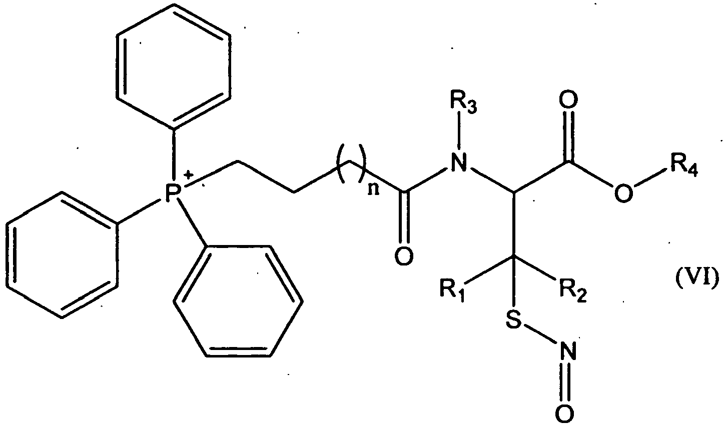 wherein n is from 0 to 27, X is an optional anion and R₁, R₂, R₃ and R₄ are independently selected from the group comprising hydrogen, alkyl and aryl.

In one embodiment n is from 0 to 20. Preferably n is from 0 to 10. More preferably n is from 1 to 5. Most preferably, n is 3.

In one embodiment R₁ R₂, R₃ and R₄ are independently selected from the group comprising hydrogen, methyl, ethyl, propyl, butyl, pentyl, hexyl and aryl. Preferably, R₁ R₂, R₃ and R₄ are methyl.

In one embodiment the anion is an inorganic anion derived from hydrochloric, hydrobromic, hydroiodic, sulfuric, sulfurous, nitric, nitrous, phosphoric or phosphorous acid, or from an alkylsulfonic or an arylsulfonic acid.

In one embodiment the anion is chloride, bromide, iodide or methanesulfonate. Preferably, the anion is bromide or methanesulfonate.

In the compound of formula (IV) the linker group comprises (C₂-C₂₉) alkylene-C(=O)-NR-C₂ alkylene substituted with alkoxycarbonyl or aryloxycarbonyl at the carbon β to the S atom and substituted with alkyl or aryl at the carbon α to the S atom.

### 3. Synthesis of compounds of the invention

The compounds of the invention can be made using any convenient synthetic process. In one embodiment the compounds of the invention can be synthesised by reacting the convenient intermediate thiol of formula (VII) with nitrous acid under an inert atmosphere as shown in Scheme 1 below.

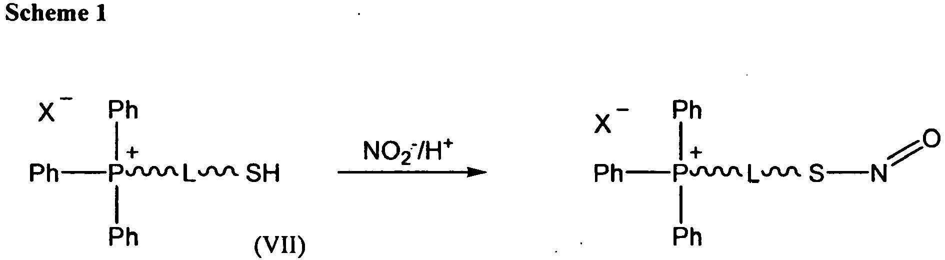

The intermediate thiol (VII) can be prepared in a number of ways depending on the final compound required. For example, in one embodiment the intermediate thiol (VII) can be prepared using Scheme 2 as outlined below (Bums, R. J., Smith, R. A. J. and Murphy, M. P. Archives of Biochemistry and Biophysics, 1995, 322, 60-68*).* 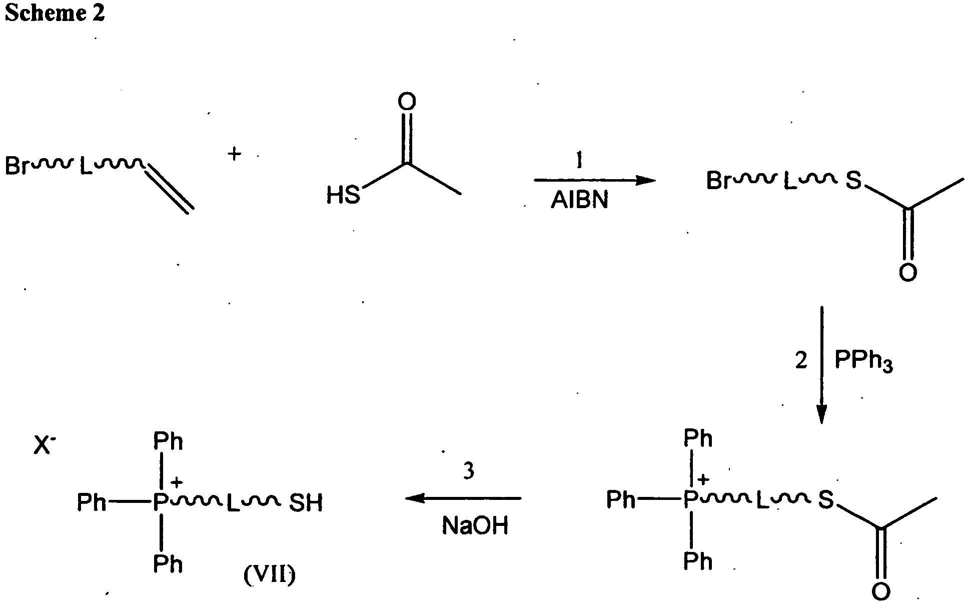
1. A linker group including a bromine group at one end and an alkylene group at another end are reacted with thioacetic acid using the radical generator 2,2'-azobisisobutyronitrile (AIBN).
2. The product of step 1 is then fused together with triphenylphosphine (PPh₃).
3. The intermediate thiol (VII) is generated by base hydrolysis with NaOH and anion exchange.

Using the synthetic schemes provided above, a number of different compounds of the invention can be prepared via nitrous acid reaction with the intermediate thiol of formula (VII). For example, Schemes 3 to 5 demonstrate how compounds of the invention incorporating substituted linker groups can be made using these synthetic schemes.

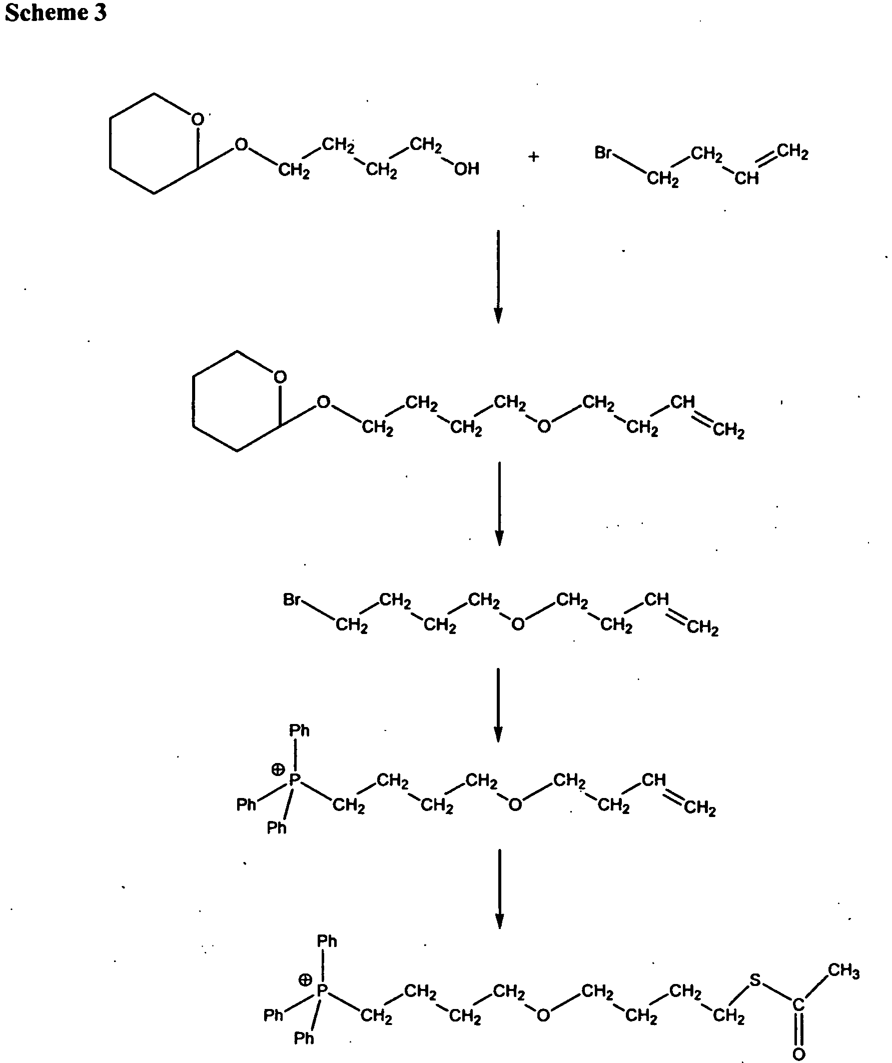

Scheme 3 outlines the synthesis of a protected thiol intermediate compound incorporating an ether functionality in the linker. Base hydrolysis provides a compound of the invention wherein the linker group is C₄ alkylene-O-C₄ alkylene.

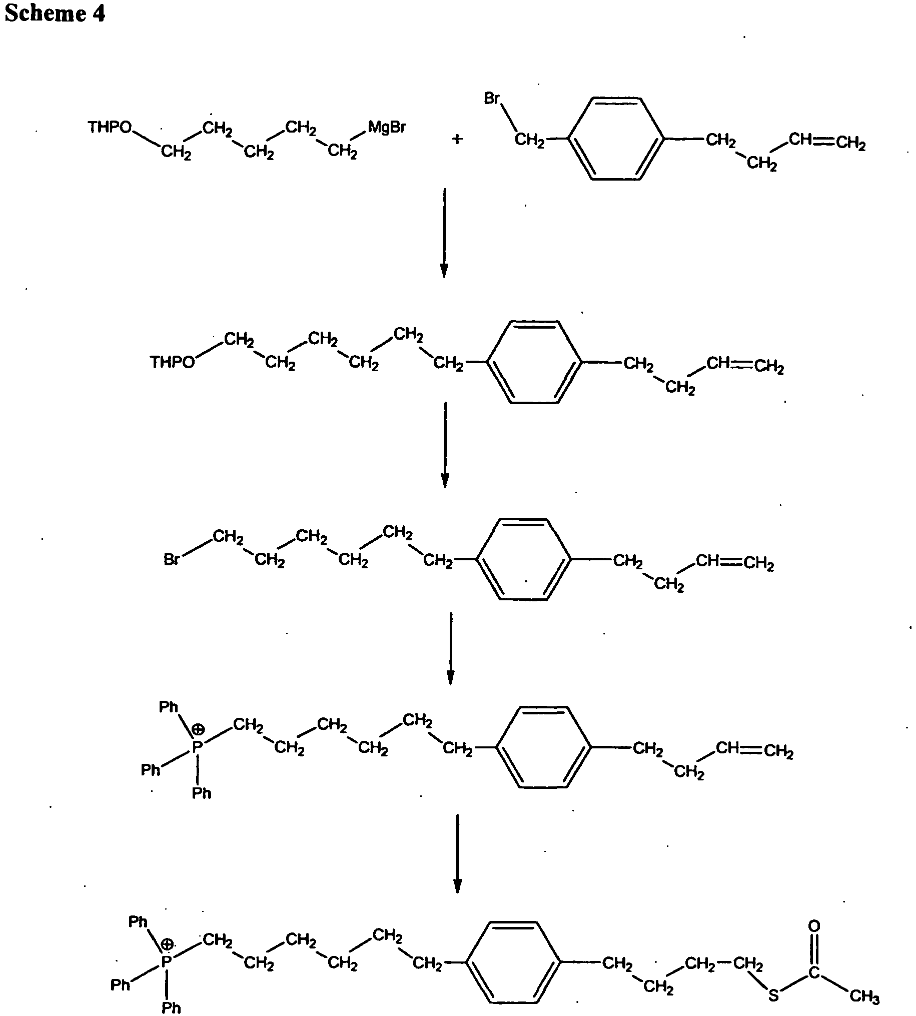

Scheme 4 outlines the synthesis of a protected thiol intermediate compound incorporating a benzene functionality in the linker. THP is tetrahydrophyran. Base hydrolysis provides a compound of the invention wherein the linker group is an aryl-containing alkylene chain, C₆ alkylene-aryl-C₄ alkylene.

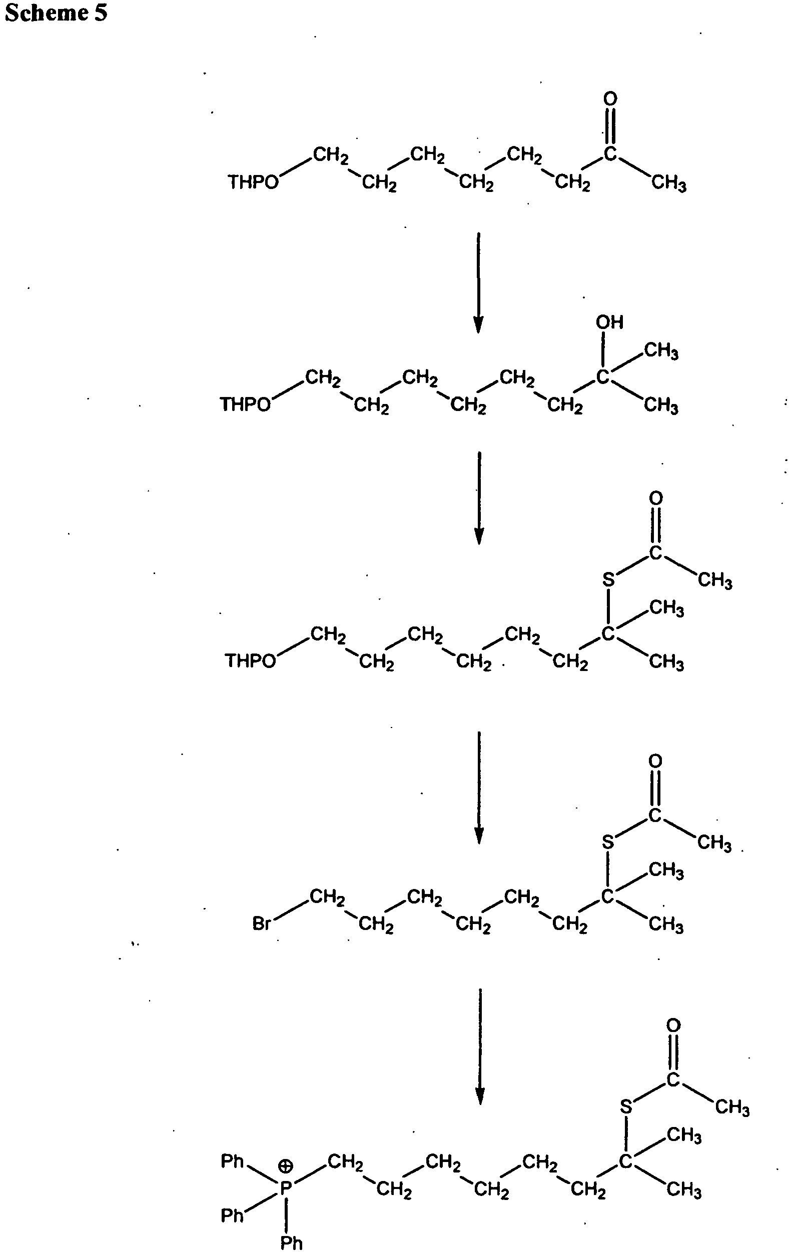

Scheme 5 outlines the synthesis of a protected thiol intermediate compound which is dimethyl substituted at the carbon α to the S atom.

Compounds of the invention including -NR-C(=O)-, -C(=O)-NR or -O-C(=O)- groups in the linker can be conveniently prepared using thietane intermediates. ' Thietanes are sulfur-containing 4-membered ring compounds.

Scheme 6 shows the synthesis of compounds of intermediate thiols that include these functionalities in the chain of the linker and are substituted with alkylcarboxlamine groups. Reaction of these intermediates with nitrous acid provides the equivalent thionitrites of the invention

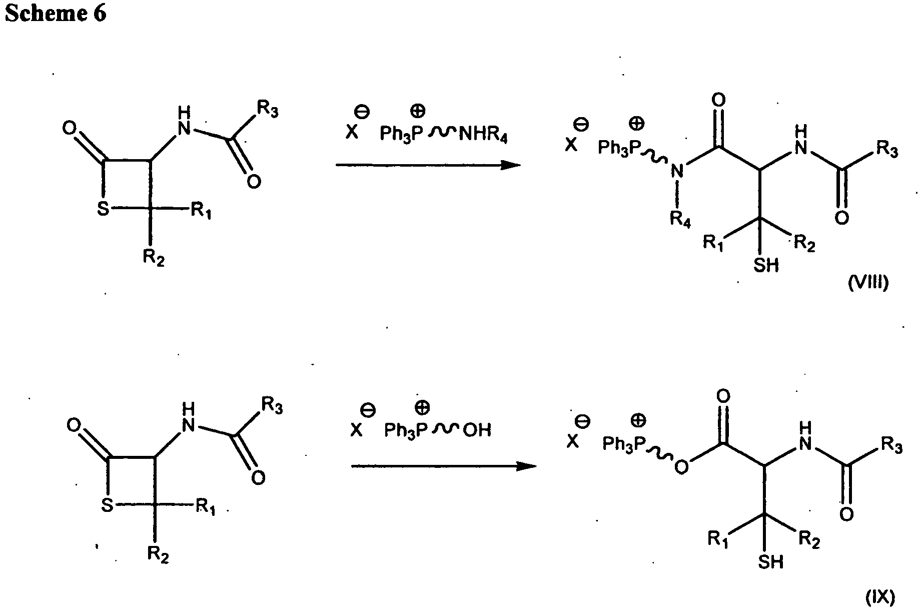

Compounds of formula (VIII) are prepared using a triphenylphosphonium cation attached to a linker group that includes an amine group at the opposite end. Compounds of formula (IX) are prepared using a triphenylphosphonium cation with a hydroxy attached to the end of the linker group. Generally the remainder of the linker group is an alkylene chain but the linker group of the thiol intermediate can be altered further by using triphenylphosphonium cations linked to the amine or hydroxy groups via other chains, for example, substituted alkylene chains.

When reacted with the thietane compound, the amine or alcohol group at the end of the linker group opens the thiotane ring to provide the thiol intermediate. If the amine group is a secondary amine (R₄ is alkyl or aryl), the resulting compound of formula (VIII) is N-alkyl or aryl substituted.

Reaction with the thietane shown above provides a thiol intermediate that is substituted with an amide group at the position β to the sulfur atom and also substituted at the carbon α to the sulfur atom. The nature of the substitution at R₁, R₂ and R₃ depends on the thietane used. Many thietanes are commercially available, for example, N-(2-ethyl-2-methyl-4-oxo-3-thietanyl) acetamide. Other alkyl and aryl groups can be introduced using thiotanes with different R₁, R₂ and R₃ substituents. Such thiotanes can easily be prepared by a person skilled in the art using known techniques.

For example, thietanes for use in synthesising compounds of the invention can also be prepared by reaction of modified cysteine, as shown in scheme 7 below.

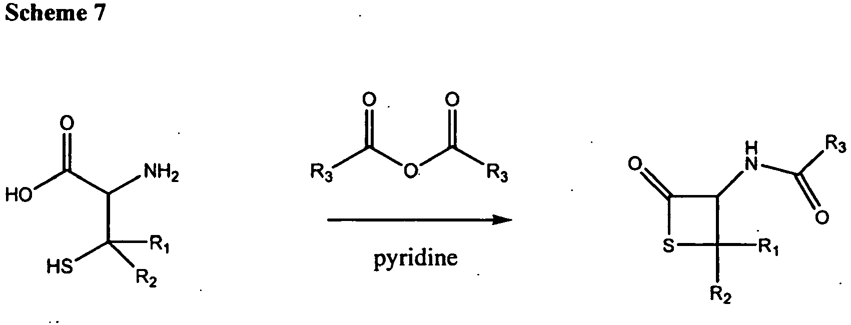

Generally, R₁, R₂ and R₃ will be alkyl or aryl.

However, if a triphenylphosphonium cation linked acid chloride is added to the reaction in Scheme 7, it is possible to make a thietane intermediate that incorporates the triphenylphosphonium cation linked functionality in the R₃ position. This compound can then be opened with an amine or alcohol to provide thiol intermediate compounds where the C(=O) is on the triphenylphosphonium end of the -C(+O)NR- group in the linker chain. Additionally, alcohol opening provides a thiol intermediate where the linker is substituted at the carbon α to the sulfur with an ester group. These reactions are shown in Scheme 8 below. 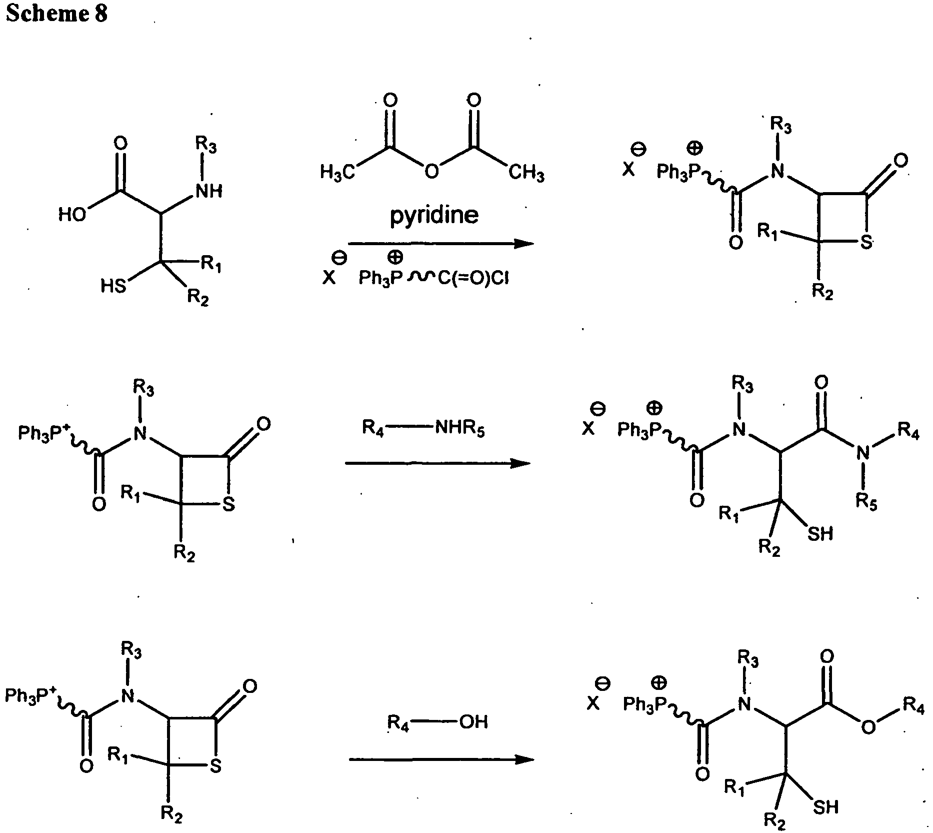 Compounds of the invention may be prepared according to the general methodology described above. It is to be understood that a skilled worker will be able, without undue experimentation and with regard to that skill and this disclosure, to select appropriate reagents and conditions to modify this methodology to produce compounds of the invention.

Those of skill in the art will also appreciate that other synthetic routes may be used to synthesize the compounds of the invention. In addition, it will be appreciated by those of skill in the art that, in the course of preparing the compounds of the invention, the functional groups of intermediate compounds may need to be protected by protecting groups. Functional groups which it is desirable to protect include, but are not limited to hydroxyl, amino and carboxylic acid. Protecting groups may be added and removed in accordance with techniques that are well known to those skilled in the art. The use of protecting groups is fully described in "Protective Groups in Organic Chemistry", edited by J.W.F McOmie, Plenum Press (1973), and "Protective Groups in Organic Synthesis", 2nd Ed, T.W. Greene and P.G.M Wutz, Wiley-Interscience (1991).

### 4. Uses of compounds of the invention

In another aspect the invention provides a pharmaceutical composition comprising a therapeutically effective amount of a compound of the invention in combination with one or more pharmaceutically acceptable excipients, carriers or diluents.

Suitable excipients, carriers and diluents can be found in standard pharmaceutical texts. See, for example, Handbook for Pharmaceutical Additives, 2nd Edition (eds. M. Ash and I. Ash), 2001 (Synapse Information Resources, Inc., Endicott, New York, USA) and Remington's Pharmaceutical Science, (ed. A. L. Gennaro) 2000 (Lippincott, Williams and Wilkins, Philadelphia, USA) which are incorporated herein by reference.

Excipients for use in the compositions of the invention include, but are not limited to microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavouring agents, colouring matter or dyes, and, if so desired, emulsifying and/or suspending agents as well, together with such diluents as water, ethanol, propylene glycol, glycerin and various like combinations thereof.

Pharmaceutical carriers include solid diluents or fillers, sterile aqueous media and various non-toxic organic solvents, and the like.

Among other effects, thionitrites protect against cellular toxicity associated with oxidative stress. This is believed to be because the majority of cellular oxidative stress originates from the mitochondria, (Murphy, M. P.; Smith, R. A. J. Advanced Drug Delivery Reviews 2000, 41, 235-250). Therefore, NO donor compounds that selectively release NO in the mitochondria have many therapeutic applications as antioxidants.

NO has been shown to be a reversible inhibitor of the mitochondrial respiratory chain by competing with oxygen at cytochrome oxidase. Selective delivery of NO to mitochondria by the compounds of the invention enables the respiration rate to be reversibly modulated within patients. Consequently, the compounds of the invention may be useful in the treatment of conditions that are affected by the inhibition of cytochrome oxidase.

The compounds of the invention do not act like other cytochrome oxidase inhibitors such as cyanide, because they compete with oxygen and thereby only affect respiration when the oxygen concentration is low. This effect is reversed once the oxygen concentration increases. Accordingly, the compounds of the invention can be considered modulators of the effective Kₘ of cytochrome oxidase.

Selective, reversible inhibition of mitochondria by the compounds of the invention can be used to treat ischaemia-reperfusion injury by preventing hypoxic tissue from becoming entirely anaerobic. Ischaemia is the condition suffered by tissues and organs when deprived of blood flow. It is mostly the result of inadequate nutrient and oxygen supply. Reperfusion injury refers to the tissue damage inflicted when blood flow is restored after an ischemic period of more than about ten minutes. Ischaemia and reperfusion can cause serious brain damage in stroke or cardiac arrest.

The compounds of the invention also have activity as angiogenesis inhibitors to prevent tumour growth and metastasis. Tumors above a certain size have to attract blood vessels to them in order to grow and blocking angiogenesis has proven to be a successful approach to targeting cancers. The signaling pathway for angiogenesis involves sensing that oxygen concentration is lowered due to poor blood supply. This is done by the oxygen dependent degradation of hypoxia inducible factor 1-α (HIF-1α). Under hypoxia, HIF-1α is no longer degraded and acts as a transcription factor inducing a range of processes including angiogenesis. It has been shown that exposure to NO can facilitate HIF-1α degradation by partially inhibiting respiration and thereby increasing the local oxygen concentration (Hagen, T., Taylor, C. T., Lam, F., Moncada, S. Science, 2003, 302, 1975-1978).

The compounds of the invention act as angiogenesis inhibitors by inhibiting cytochrome oxidase thereby increasing the local oxygen concentration in hypoxic tumours. This destabilises the transcription factor hypoxia inducible factor 1-α (HIF-1α) thereby blocking angiogenesis to the tumour.

Thus, the selective production of NO within mitochondria by the compounds of the invention may act to switch off angiogenesis in tumors without affecting respiration in fully aerobic tissues. This will result in a slowing of tumour development.

Nitric oxide has also been implicated in the neurodegeneration pathology of Alzheimer's and Parkinson's diseases (Stamler, J.S., Jaraki, O., Osborne, J., Simon, D.I., Keaney, J., Vita, J., Singel, D., Valeri, C.R., Loscalzo, J. Proceedings of the National Academy-of Sciences of the United States of America,1992, 89, pp 7674-7.

NO is thought to affect these disorders via interactions with cytochrome c oxidase and complex I respectively. Therefore, the NO donor compounds of the invention may have therapeutic application in the treatment and management of Alzheimer's and Parkinson's diseases.

The compounds of the invention can also be used in conjunction with other therapies such as anti cancer agents and other pharmaceuticals.

The conjugates or pharmaceutical compositions of the invention can be administered via oral, parenteral (such as subcutaneous, intravenous, intramuscular, intracisternal and infusion techniques), rectal, intranasal or topical routes. In general, these compounds are administered in doses ranging from about 0.5 to about 500 mg per day, in single or divided doses (such as from 1 to 4 doses per day).

It will be appreciated by one of skill in the art that appropriate dosages of the compounds, and compositions comprising the compounds, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition and the general health and prior medical history of the patient.

The active compounds of this invention can be administered alone or in combination with pharmaceutically acceptable excipients, carriers or diluents by any of the routes previously indicated, and such administration may be carried out in single or multiple doses.

More particularly, the novel therapeutic agents of this invention can be administered in a wide variety of different dosage forms, they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions injectable solutions, elixirs, syrups, and the like. Such carriers include solid diluents or fillers, sterile aqueous media and various non-toxic organic solvents, and the like. Injectable forms are preferred.

Moreover, oral pharmaceutical compositions can be suitably sweetened and/or flavoured. In general, the conjugates of the invention are present in such dosage forms at concentration levels ranging from about 1.0% to about 70% by weight, preferably about 1.0% to about 70% by weight.

For oral use in treating the various disorders and conditions referred to above, the conjugates can be administered, for example, in the form of tablets or capsules, or as an aqueous solution or suspension. Tablets may contain various excipients such as described above.

For parenteral administration, solutions of a compound of the present invention in oils such as sesame or peanut oil, or an aqueous propylene glycol may be employed. The aqueous solutions should be suitably buffered (preferably pH less than 8) if necessary and the liquid diluent first rendered isotonic. These aqueous solutions are suitable for intravenous injection purposes. The oily solutions are suitable for intra-muscular and subcutaneous injection purposes. The preparation of all these solutions under sterile conditions is readily accomplished by standard pharmaceutical techniques well known to those skilled in the art.

For intramuscular, parenteral and intravenous use, sterile solutions of the active ingredient can be prepared, and the pH of the solutions should be suitably adjusted and buffered. For intravenous use, the total concentration of solutes should be controlled to render the preparation isotonic.

Various aspects of the invention will now be illustrated in non-limiting ways by reference to the following examples.

### EXAMPLE 1

A compound of formula (X) was prepared as described in Schemes 1 and 2 above.

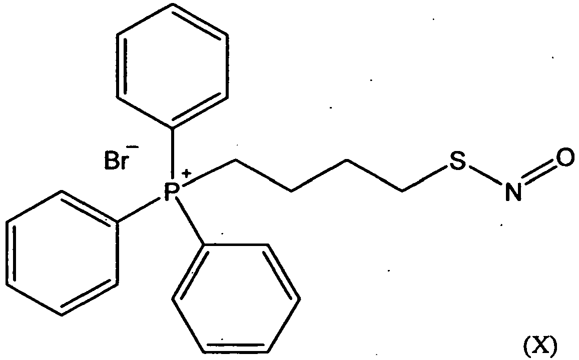

All solutions were sparged with argon for 60 sec before use. 4-Thiobutyltriphenylphosphonium bromide (25.4 mg, 56.8 µmol) was dissolved in ethanol (0.300 mL) and the solution was flushed with argon and cooled on ice in the dark, for 10 min. Aqueous hydrochloric acid (0.130 mL, 80.6 µmol) was added and the reaction vessel swirled in the ice and then 1 min later an aqueous solution of sodium nitrite (0.120 mL, 92.3 µmol) was added and the mixture went bright pink. After standing 30 min on ice the product was extracted with chloroform (1 mL). The organic solution was washed to 10% aqueous sodium bromide (1 mL), dried over anhydrous MgSO₄ (~50 mg) and kept in the dark at -20°C until required. The solvents were removed *in vacuo* to give red residue of the compound of formula (X). ³¹P NMR (chloroform-d₁) δ 1.867 compared with ethyltriphenylphosphonium bromide reference(δ = 0 ppm).

The compound, herein referred to as MitoSNO, was found to have a high resolution mass spectrum of 380.1238 (calc. for C₂₂H₂₃PSNO is 380.1). The compound was also found to have an extinction coefficient of 16 L mol⁻¹ cm⁻¹ (at λ=549 nm).

### EXAMPLE 2

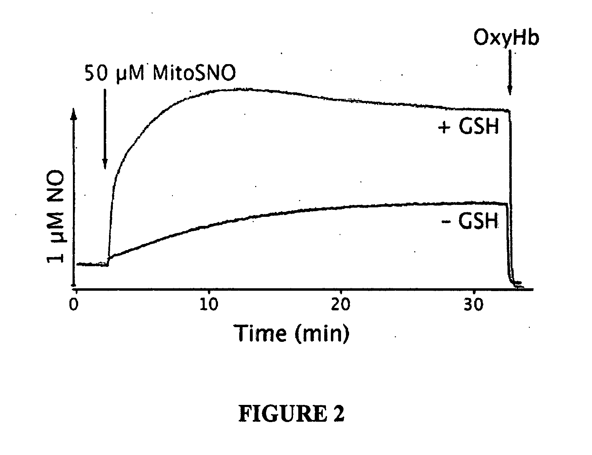

An NO-selective electrode was used to demonstrate NO release from MitoSNO. 50 µM MitoSNO was incubated ± 10 mM GSH in KPi buffer, pH 8, at 37°C for 30 min, then oxyhaemoglobin (5 µM) was added at the end of the incubation to degrade the free NO to nitrate (NO₃⁻) and return the electrode response to the baseline. This showed that MitoSNO releases NO both in the presence and absence of GSH as can be seen in Figure 2.

### EXAMPLE 3

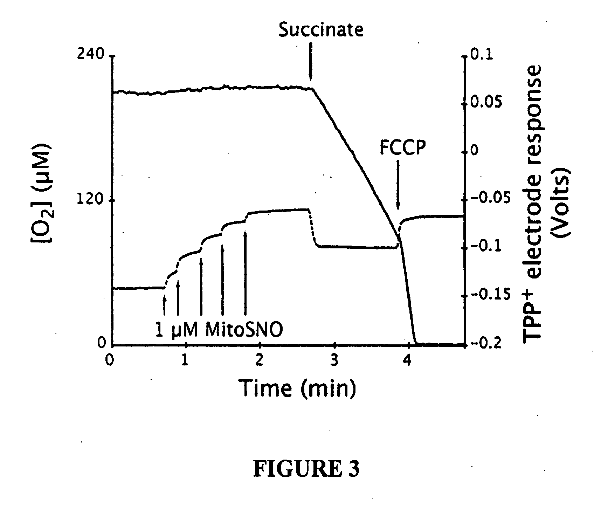

Rat liver mitochondria (1 mg protein/ml) in KCl buffer was incubated at 37°C with 5 µM MitoSNO. 1 µM additions of MitoSNO were made sequentially before addition of 10 mM succinate. 200 nM FCCP was then added to uncouple the mitochondria. The concentrations of O₂ and MitoSNO in solution were measured simultaneously using a Clark-type O₂ electrode in combination with an ion-selective electrode for triphenylphosphonium cation (TPP⁺). MitoSNO was found to be taken up by isolated mitochondria and inhibited respiration by NO release. This can be seen in Figure 3.

When succinate was added, the mitochondria started respiring and set up a Δψ, which caused a decrease in the MitoSNO concentration, as detected by the ion-selective electrode (Figure 3). When the mitochondria were uncoupled by FCCP, a concomitant increase in the concentration of MitoSNO, or its derivatives, in the medium was detected by the ion-selective electrode. The amount of MitoSNO taken up into the mitochondria from the trace in Figure 3 corresponds to ~2.5 µM of the initial external concentration of 5 µM. Therefore 7.5 nmol MitoSNO have been taken up into 3 mg protein, and as the mitochondrial volume is - 0.5 µl/mg protein (Brown, G. C. and Brand, M. D. Biochem J., 1985, 225, 399-405), the concentration of MitoSNO within mitochondria is - 5 mM. This represents a 2000 fold concentration relative to the 2.5 µM present in the external medium, and is consistent with a Nernstian uptake of MitoSNO.

### EXAMPLE 4

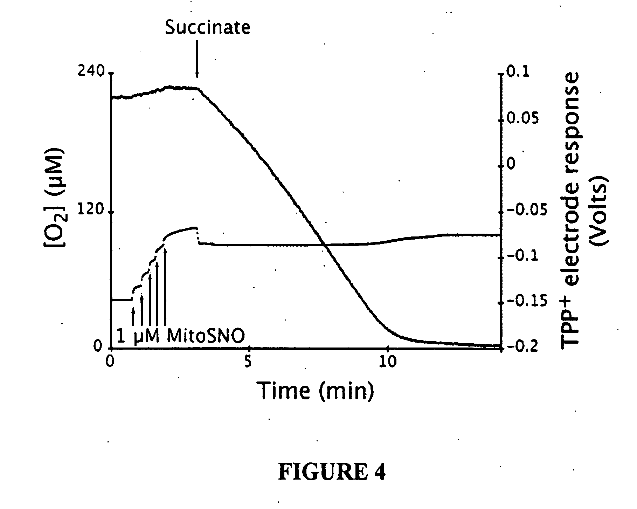

To show that MitoSNO released NO within energized mitochondria and inhibited mitochondrial respiration, 5 µM MitoSNO was incubated with a low concentration of rat liver mitochondria (0.5 mg protein/ml) in KCl buffer at 37°C, to enable a longer incubation period in order for sufficient NO release from MitoSNO to affect respiration. 1 µM additions of MitoSNO were made sequentially before addition of 10 mM succinate. The mitochondria were then allowed to respire until oxygen consumption reached a plateau. At low oxygen concentrations, inhibition of respiration and a concomitant gradual redistribution of MitoSNO, or its derived TPP cations, to the extramitochondrial solution through decreased Δψ were observed (Figure 4).

### EXAMPLE 5

A compound of formula (XI) was prepared as described in Scheme 6 above.

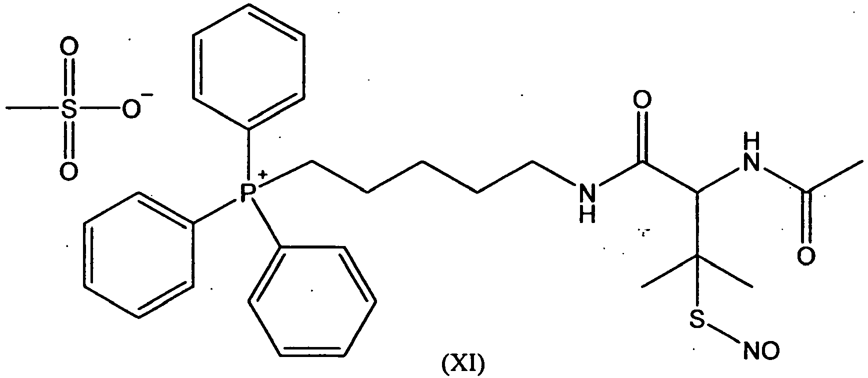

A solution of 5-aminopentyltriphenylphosphonium bromide hydrogen bromide (McAllister, P. R.; Dotson, M. J.; Grim, S. O.; Hillman, G. R. Journal of Medicinal Chemistry 1980, 23, pp 862-5) (467 mg, 0.917 mmol) and triethylamine (136 µL, 0.920 mmol) in dichloromethane (10 mL) was stirred for 5 min after which time a solution of N-(2,2-Dimethyl-4-oxo-thietan-3-yl)-acetamide (Moynihan, H. A., Roberts, S. M. Journal of the Chemical Society, Perkin Transactions 1: Organic and Bio-Organic Chemistry (1972-1999) 1994, pp 797-805) (157 mg, 0.917 mmol) in dichloroinethane (20 mL) was added in one aliquot and the reaction mixture was stirred for a further 4 h. After the mixture had been washed with a saturated solution of sodium methanesulfonate (3 × 20 mL), the organic fraction was collected, dried (MgSO₄), filtered and the solvents removed *in vacuo* to give pale yellow oil. The oil was dissolved in minimal dichloromethane (2 mL) and then excess diethyl ether (70 mL) was added. After the resultant precipitate had settled, the solvents were decanted and the residue was dried *in vacuo* (0.1 mmHg) for 2 h to give a thiol as a cream powder (374 mg, 0.607 mmol, 66%). HRPI ESMS (CE) calculated for C₃₀H₃₈N₂O₂PS⁺: 521.2386, found: 521.2397. Analysis calculated for C₃₁H₄₁N₂O₅PS₂: C 60.4, H 6.7, N 4.5, S 10.4%; found C 59.8, H 6.9, N 4.8, S 10.2%. UV-vis: 2.7 mg in 10 mL ethanol, ε₂₆₈ 2660 Lmol⁻¹cm⁻¹, ε₂₇₅ 2292 Lmol⁻¹cm⁻¹.¹H NMR (chloroform-d₁) δ 7.94 (1H, t, *J* = 5 Hz, CH₂-N**H**), 7.82-7.62 (16H, m, Ar**H**, CH-N**H**), 4.67 (1H, d, *J*= 10 Hz, C**H**), 3.58-3.47 (2H, m, CH₂-P⁺), 3.42-3.27 (1H, m, CH**H**-NH), 3.27-3.14 (1H, m, C**H**H-NH), 2.76 (3H, s, SO₃-C**H**₃), 2.69 (1H, s, S**H**), 2.13 (3H, s, CO-C**H**₃), 1.55 (3H, s, C**H**₃), 1.43 (3H, s, C**H**₃), 1.80-1.52 (6H, m, C**H**₂ ppm. ¹³C NMR (chloroforin-d₁, 125 MHz) δ170.7, (**C**O), 170.3, (**C**O), 135.1 (d, *J*= 3 Hz, *para*-Ph), 133.6 (d, *J* = 10 Hz, *meta*-Ph), 130.6 (d, *J* = 13 Hz, *ortho*-Ph), 118.5 (d, *J* = 86 Hz, *ipso*-Ph), 62.3 (CH), 46.5 (**C**), 39.8 (**C**H₃-SO₃⁻), 38.2 (**C**H₂-NH), 30.7 (**C**H₃), 30.6, (**C**H₃), 27.6 (**C**H₂-CH₂-NH), 27.1 (d, *J* = 17 Hz, **C**H₂-CH₂-P⁺), 23.4 (**C**H₃), 22.0 (d, *J* = 53 Hz, **C**H₂-P⁺), 21.9 (d, *J* = 5 Hz, **C**H₂-CH₂-CH₂-P⁺) ppm. ³¹P NMR (chloroform-d₁) δ 25.4 ppm. HPLC: mobile phase 50% acetonitrile, 50% water (0.1% trifluoroacetic acid); Prodigy 5µ OD53 11A 250×4 mm, detection 275 nm, flow rate 1.00 mL/min; retention time 4.76 min, one peak.

A sample of the thiol (50 mg, 0.081 mmol) was dissolved in ethanol (2 mL) flushed with argon and stored in the dark. Methane sulfonic acid stock (0.576 mL, 357 mmol) was added and the reaction vessel swirled for 1 min then aqueous sodium nitrite (0.420 mL, 323 mmol) was added and the reaction vessel was swirled again. The reaction mixture was held in the dark for 2 hr at room temperature. Dichloromethane (2 mL) was then added and the mixture was gently shaken for 10 sec. Distilled water (2 mL) was added and the reaction mixture was left to stand for 20 sec and the lower dichloromethane layer was removed and dried over anhydrous MgSO₄ (- 200 mg). The dichloromethane solution was then filtered and evaporated (30°C, 2 mmHg) The green glassy residue was dissolved in minimal dichloromethane (0.5 mL) and added to diethyl ether(40 mL). The resulting pale green precipitate was isolated by decantation and drying under vacuum (2 mmHg) and in the dark to yield the product (V) (40 mg, 78%). HPLC. 50% acetonitrile, 50% water (0.1% trifluoroacetic acid); Prodigy 5µ OD53 11A 250×4 mm, detection 275 nm, flow rate 1.00 mL/min; retention time 8.17 min, one peak. UV-vis: ethanol λₘₐₓ = 268 nm (ε 3191 Lmol^{- 1}cm⁻¹), λ = 275 nm (ε 2730 Lmol⁻¹cm⁻¹), λ = 344 nm (ε 601 Lmol⁻¹cm⁻¹). HRPI ESMS (CE) (CE 6V, QIE 3V) calculated for C₃₀H₃₇N₃O₃PS: 550.229, found: 550.226. ¹H NMR δ (methanol-d₄): 8.38 (1H, bt, CH₂-N**H**), 7.73-7.92 (15H, m, Ar-**H**), 5.30 (1H, d, *J*= 10 Hz, C**H**), 3.47-3.35 (2H, m, P⁺-C**H**₂), 3.22-3.07 (2H, m, C**H**₂-NH), 2.68 (3H, s, C**H**₃-SO₃⁻), 2.03 (3H, s, C**H**₃), 1.99 (3H, s, C**H**₃), 1.96 (3H, s, C**H**₃), 1.88-1.40 (6H, m, C**H**₂) ppm. ¹³C NMR δ (methanol-d₄, 125 MHz): 173.1 (**C**O), 170.8 (**C**O), 136.3 (d, *J*=3 Hz, *para*-Ph), 134.8 (d, *J*= 10 Hz, *meta*-Ph)*,* 131.5 (d, *J* = 13 Hz, *ortho*-Ph)*,* 119.9 (d, *J* = 86 Hz, *ipso*-Ph), 61.7 (**C**H), 59.2 (**C**), 39.8 (**C**H₂-NH), 39.5 (**C**H₃-SO₃⁻), 29.3 (**C**H₂-CH₂-NH), 28.7 (d, *J*= 17 Hz, **C**H₂-CH₂-P⁺), 27.1, (**C**H₃), 25.9, (**C**H₃), 23.0 (d, *J*= 9 Hz, **C**H₂-CH₂-CH₂-P⁺), 22.7 (d, *J*= 56 Hz, **C**H₂-P⁺), 22.4 (**C**H₃) ppm. ³¹P NMR λ (methanol-d₄) 24.9 ppm. This compound, [5-(2-acetylamino-3-methyl-3-nitrosothio-butyrylamino)-pentyl]-triphenylphosphonium methanesulfonate, is herein referred to as MitoSNAP.

### EXAMPLE 6

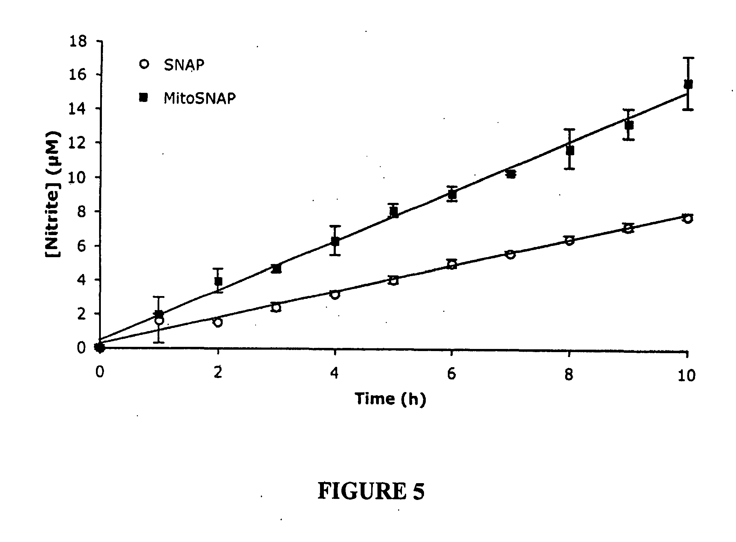

To show that MitoSNAP could spontaneously release NO, S-nitroso-N-acetyl-DL-penicillamine (SNAP) (50 µM) or MitoSNAP (50 µM) was incubated at 37°C in opaque-tubes in KCl buffer (120 mM KCl, 10 mM HEPES, 1 mM Ethylene-bis(oxyethylenenitrilo)tetraacetic acid (EGTA)) with 100 µm Diethylenetriaminepentaacetic acid (DTPA) and Neocuproine (10 µM) that had been treated with Chelex (1%). Samples were taken hourly and snap frozen on dry ice. Samples were thawed and assayed for nitrite concentration using the Griess assay. Data are means of triplicate determinations of a single experiment that was repeated 3x with similar results (Figure 5).

### EXAMPLE 7

To show that MitoSNAP could release NO, nitrogen purged KCl buffer (as above) at 37°C was incubated in the stirred chamber of a nitric oxide electrode (WPI Ltd). MitoSNAP (100 µM) was added and the formation of NO measured by the NO electrode. Where indicated oxyhaemoglobin (~5µM) was added to degrade all the accumulated NO to nitrate.

### EXAMPLE 8

To see the interaction of MitoSNAP with mitochondria, rat liver mitochondria (1 mg protein /ml) were incubated KCl buffer (as above) supplemented with rotenone (4µg/ml) +/- FCCP (0.5µM) at 37°C and the oxygen and NO concentrations were measured using appropriate electrodes. For the incubation without FCCP, MitoSNAP (50µM) was added followed by succinate (10mM) which induced a mitochondrial membrane potential leading to the uptake of MitoSNAP inside mitochondria which led to its activation by thiols within mitochondria and to the release of large amounts of NO. This NO led to the inhibition of respiration at cytochrome oxidase and this was reversed by the addition of oxyhaemoglobin (~5µM) to degrade all accumulated NO to nitrate.

### INDUSTRIAL APPLICABILITY

The compounds of the invention provide a delivery system for NO directly into the mitochondria. The compounds are selectively taken up by the mitochondria driven by the membrane potential. Selective delivery of NO in the mitochondria allows targeting of local NO-sensitive variables, such as the inhibition of respiration. The compounds of the invention can also S-nitosylate proteins present in the mitochondria. Conventional NO donors produce NO throughout the cell and affect a great number of NO signaling pathways.

The compounds of the invention are of use in the treatment of conditions which are affected by mitochondrial respiration such as ischaemia-reperfusion injury. They are also able to inhibit angiogenesis and therefore are of use in the treatment and prevention of tumour growth and/or metastasis.

## Claims

1. A compound of formula (I) 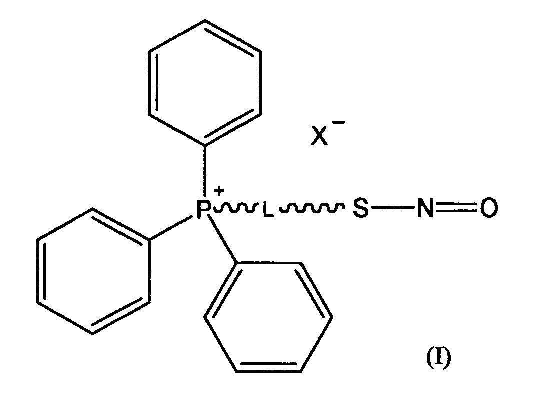 wherein -L- is a linker group and X is an optional anion; and
wherein the linker group is selected from the group comprising
(a) (C₁-C₃₀) alkylene,
(b) (C₁-Cₓ) alkylene-NR-(C₁-C_{y}) alkylene, wherein R is H, alkyl, or aryl,
(c) (C₁-Cₓ) alkylene-NR-C(=O)-(C₁-C_{y}) alkylene, wherein R is H, alkyl, or aryl,
(d) (C₁-Cₓ) alkylene-C(=O)-NR-(C₁-C_{y}) alkylene, wherein R is H, alkyl, or aryl,
(e) (C₁-Cₓ) alkylene-O-(C₁-C_{y}) alkylene,
(f) (C₁-Cₓ) alkylene-O-C(=O)-(C1-C_{y}) alkylene,
(g) (C₁-Cₓ) alkylene-S-(C₁-C_{y}) alkylene, and
(h) (C₁-Cₓ) alkylene-aryl-(C₁-C_{y}) alkylene,
wherein x + y = 30 and wherein alkylene is optionally substituted with one or more functional groups independently selected from the group consisting of hydrogen, alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, oxy, amino, alkylamino, aminocarbonyl, alkoxycarbonyl, aryloxycarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl, or the substituent groups of adjacent carbon atoms in the linker group can be taken together with the carbon atoms to which they are attached to form a carbocycle or a heterocycle.

2. A compound of claim 1 wherein the linker group is selected from the group comprising
(a) (C₁-C₃₀) alkylene,
(b) (C₁-Cₓ) alkylene-NR-C(=O)-(C₁-C_{y}) alkylene, wherein R is H, alkyl, or aryl,
(c) (C₁-Cₓ) alkylene-C(=O)-NR-(C₁-C_{y}) alkylene, wherein R is H, alkyl, or aryl, and
(d) (C₁-Cₓ) alkylene-O-C(=O)-(C₁-C_{y}) alkylene,
wherein x + y = 30, wherein alkylene is optionally substituted with one or more functional groups independently selected from the group consisting of alkyl, cycloalkyl, alkenyl, alkynyl, haloalkyl, aryl, aminoalkyl, hydroxyalkyl, alkoxyalkyl, alkylthio, alkylsulfinyl, alkylsulfonyl, carboxyalkyl, cyano, oxy, amino, alkylamino, aminocarbonyl, alkoxycarbonyl, aryloxycarbonyl, alkylaminocarbonyl, arylaminocarbonyl, aralkylaminocarbonyl, alkylcarbonylamino, arylcarbonylamino, aralkylcarbonylamino, alkylcarbonyl, heterocyclocarbonyl, aminosulfonyl, alkylaminosulfonyl, alkylsulfonyl, and heterocyclosulfonyl, or the substituent groups of adjacent carbon atoms in the linker group can be taken together with the carbon atoms to which they are attached to form a carbocycle or a heterocycle.

3. A compound of claim 2 wherein the linker group is selected from the group comprising
(a) (C₁-C₁₂) alkylene,
(b) (C₁-C₇) alkylene-NR-C(=O)-(C₁-C₅) alkylene, wherein R is H, alkyl, or aryl,
(c) (C₁-C₇) alkylene-C(=O)-NR-(C₁-C₅) alkylene, wherein R is H, alkyl, or aryl, and
(d) (C₁-C₇) alkylene-O-C(=O)-(C₁-C₅) alkylene,
wherein alkylene is optionally substituted with one or more functional groups independently selected from the group consisting of alkyl, aryl, alkoxycarbonyl, aryloxycarbonyl, alkylaminocarbonyl, arylaminocarbonyl, alkylcarbonylamino, and arylcarbonylamino.

4. A compound of any one of claims 1 to 3 wherein alkylene is substituted at the carbon β to the sulfur atom with one or more functional groups independently selected from the group consisting of alkoxycarbonyl, alkylaminocarbonyl and alkylcarbonylamino.

5. A compound of any one of claims 1 to 4 wherein alkylene is substituted at the carbon α to the sulfur atom with alkyl or aryl.

6. A compound of any one of claims 1 to 5 wherein the optional anion is an anion derived from an acid selected from the group comprising hydrochloric, hydrobromic, hydroiodic, sulfuric, sulfurous, nitric, nitrous, phosphoric, phosphorous, alkylsulfonic or arylsulfonic acid.

7. A compound of claim 1 wherein the compound is selected from the group comprising 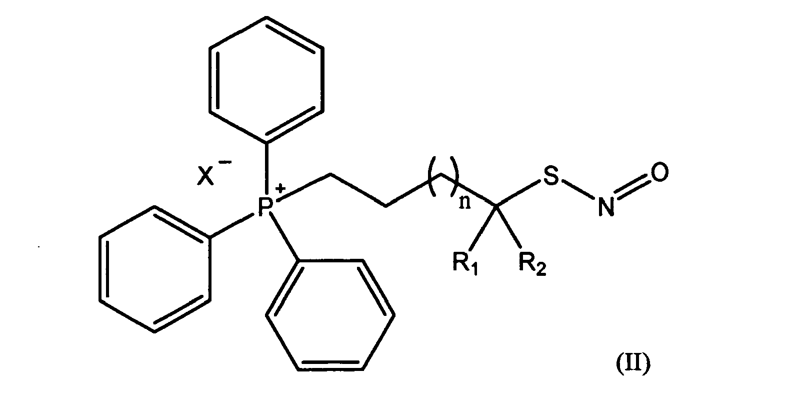 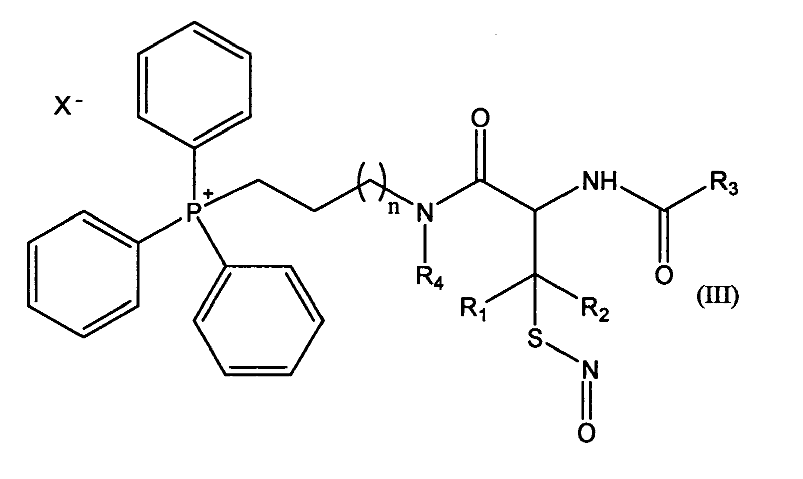 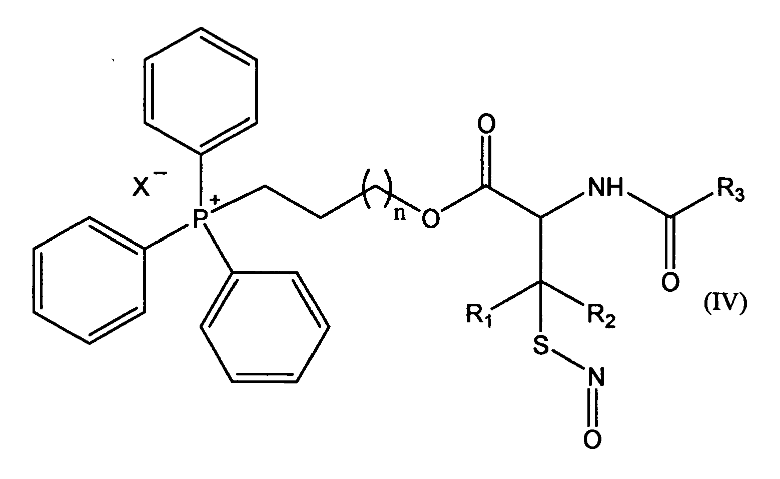 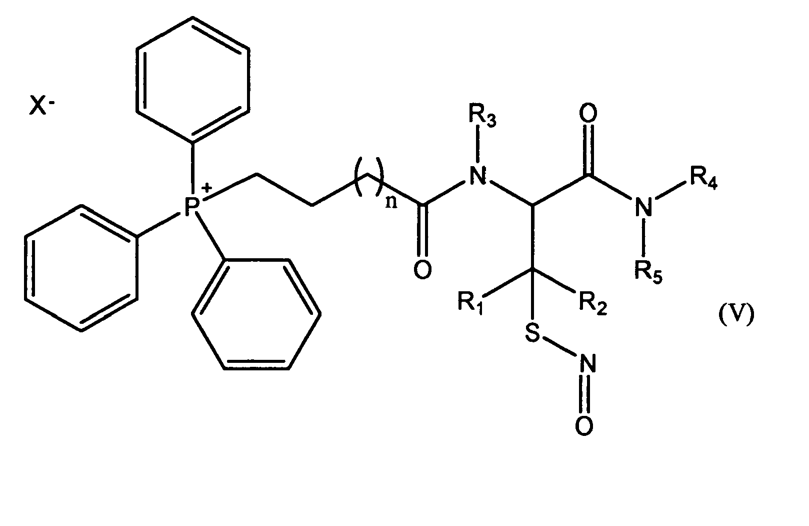 or 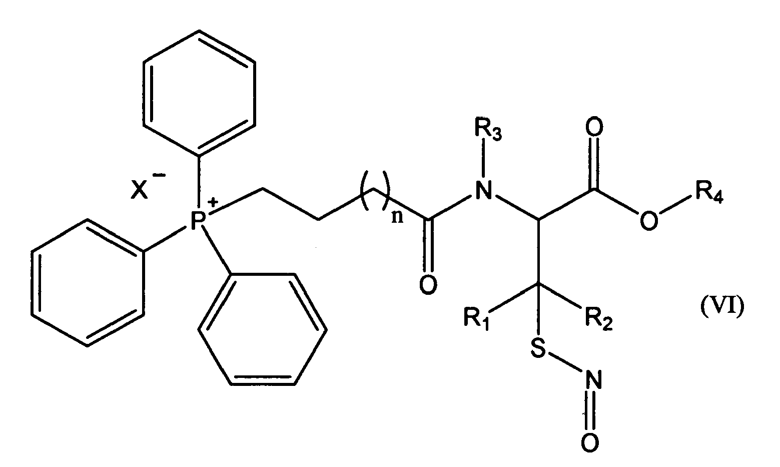 wherein n is from 0 to 27, X is an optional anion and R₁ and R₂ are independently selected from the group comprising hydrogen, alkyl and aryl.

8. A compound of claim 1 selected from the group comprising

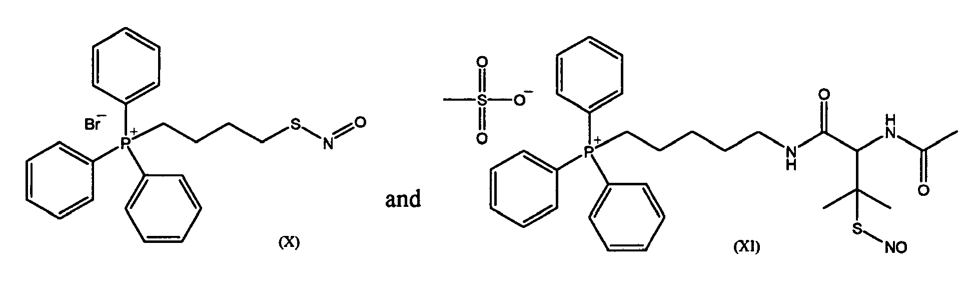

9. A pharmaceutical composition comprising a therapeutically effective amount of a compound of any one of claims 1-8 with one or more pharmaceutically acceptable excipients, carriers or diluents.

10. A use of a compound of any one of claims 1-8 in the preparation of a medicament for the treatment of a disease or disorder selected from the group comprising cancer, neoplasms, tumor growth, metastasis, angina, stroke, myocardial infarction and ischaemia-reperfusion injury.

11. A use of a compound of any one of claims 1-8 in the preparation of a medicament for inhibiting angiogenesis in a subject.

12. A use of a compound of any one of claims 1-8 in the preparation of a medicament for generating NO in the mitochondria of a subject.

13. A use of a compound of any one of claims 1-8 in the preparation of a medicament for inhibiting cytochrome oxidase in the mitochondria of a subject.

14. A use of a compound of any one of claims 1-8 in the preparation of a medicament for S-nitrosylating proteins in the mitochondria of a subject.

## Patentansprüche

1. Verbindung der Formel (I) 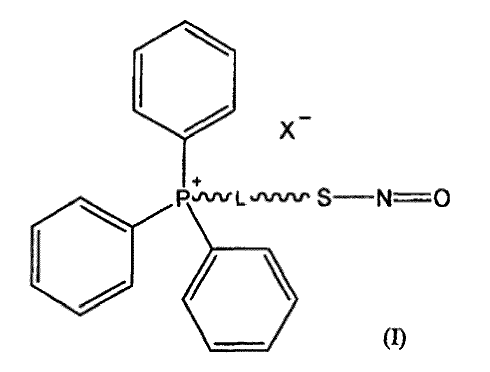 worin ~L~ eine Linkergruppe ist und X ein optionales Anion ist; und
worin die Linkergruppe aus der aus
(a) (C₁-C₃₀)-Alkylen,
(b) (C₁-Cₓ)-Alkylen-NR-(C₁-C_{y})-alkylen, worin R = H, Alkyl oder Aryl ist,
(c) (C₁-Cₓ)-Alkylen-NR-C(=O)-(C₁-C_{y})-alkylen, worin R = H, Alkyl oder Aryl ist,
(d) (C₁-Cₓ)-Alkylen-C(=O)-NR-(C₁-C_{y})-alkylen, worin R = H, Alkyl oder Aryl ist,
(e) (C₁-Cₓ)-Alkylen-O-(C₁-C_{y})-alkylen,
(f) (C₁-Cₓ)-Alkylen-O-C(=O)-(C₁-C_{y})-alkylen,
(g) (C₁-Cₓ)-Alkylen-S-(C₁-C_{y})-alkylen und
(h) (C₁-Cₓ)-Alkylenaryl-(C₁-C_{y})-alkylen
bestehenden Gruppe ausgewählt ist, worin x + y = 30 ist und worin Alkylen gegebenenfalls mit einer oder mehreren, jeweils unabhängig aus der aus Wasserstoff, Alkyl, Cycloalkyl, Alkenyl, Alkinyl, Halogenalkyl, Aryl, Aminoalkyl, Hydroxyalkyl, Alkoxyalkyl, Alkylthio, Alkylsulfinyl, Alkylsulfonyl, Carboxyalkyl, Cyano, Oxy, Amino, Alkylamino, Aminocarbonyl, Alkoxycarbonyl, Aryloxycarbonyl, Alkylaminocarbonyl, Arylaminocarbonyl, Aralkylaminocarbonyl, Alkylcarbonylamino, Arylcarbonylamino, Aralkylcarbonylamino, Alkylcarbonyl, Heterocyclocarbonyl, Aminosulfonyl, Alkylaminosulfonyl, Alkylsulfonyl und Heterocyclosulfonyl bestehenden Gruppe ausgewählten funktionellen Gruppen substituiert ist oder gegebenenfalls die Substituentengruppen benachbarter Kohlenstoffatome in der Linkergruppe zusammen mit den Kohlenstoffatomen, an die sie gebunden sind, einen Carbozyklus oder einen Heterozyklus bilden.

2. Verbindung nach Anspruch 1, worin die Linkergruppe aus der aus
(a) (C₁-C₃₀)-Alkylen,
(b) (C₁-Cₓ)-Alkylen-NR-C(=O)-(C₁-C_{y})-alkylen, worin R = H, Alkyl oder Aryl ist,
(c) (C₁-Cₓ)-Alkylen-C(=O)-NR-(C₁-C_{y})-alkylen, worin R = H, Alkyl oder Aryl ist,
und
(d) (C₁-Cₓ)-Alkylen-O-C(=O)-(C₁-C_{y})-alkylen
bestehenden Gruppe ausgewählt ist, worin x + y = 30 ist und worin Alkylen gegebenenfalls mit einer oder mehreren, jeweils unabhängig aus der aus Wasserstoff, Alkyl, Cycloalkyl, Alkenyl, Alkinyl, Halogenalkyl, Aryl, Aminoalkyl, Hydroxyalkyl, Alkoxyalkyl, Alkylthio, Alkylsulfinyl, Alkylsulfonyl, Carboxyalkyl, Cyano, Oxy, Amino, Alkylamino, Aminocarbonyl, Alkoxycarbonyl, Aryloxycarbonyl, Alkylaminocarbonyl, Arylaminocarbonyl, Aralkylaminocarbonyl, Alkylcarbonylamino, Arylcarbonylamino, Aralkylcarbonylamino, Alkylcarbonyl, Heterocyclocarbonyl, Aminosulfonyl, Alkylaminosulfonyl, Alkylsulfonyl und Heterocyclosulfonyl bestehenden Gruppe ausgewählten funktionellen Gruppen substituiert ist oder gegebenenfalls die Substituentengruppen benachbarter Kohlenstoffatome in der Linkergruppe zusammen mit den Kohlenstoffatomen, an die sie gebunden sind, einen Carbozyklus oder einen Heterozyklus bilden.

3. Verbindung nach Anspruch 2, worin die Linkergruppe aus der aus
(a) (C₁-C₁₂)-Alkylen,
(b) (C₁-C₇)-Alkylen-NR-C(=O)-(C₁-C₅)-alkylen, worin R = H, Alkyl oder Aryl ist,
(c) (C₁-C₇)-Alkylen-C(=O)-NR-(C₁-C₅)-alkylen, worin R = H, Alkyl oder Aryl ist, und
(d) (C₁-C₇)-Alkylen-O-C(=O)-(C₁-C₅)-alkylen bestehenden Gruppe ausgewählt ist, worin Alkylen gegebenenfalls mit einer oder mehreren, jeweils unabhängig aus der aus Alkyl, Aryl, Alkoxycarbonyl, Aryloxycarbonyl, Alkylaminocarbonyl, Arylaminocarbonyl, Alkylcarbonylamino und Arylcarbonylamino bestehenden Gruppe ausgewählten funktionellen Gruppen substituiert ist.

4. Verbindung nach einem der Ansprüche 1 bis 3, worin Alkylen am Kohlenstoff β zum Schwefelatom mit einer oder mehreren, jeweils unabhängig aus der aus Alkoxycarbonyl, Alkylaminocarbonyl und Alkylcarbonylamino bestehenden Gruppe ausgewählten funktionellen Gruppen substituiert ist.

5. Verbindung nach einem der Ansprüche 1 bis 4, worin Alkenyl am Kohlenstoff α zum Schwefelatom mit Alkyl oder Aryl substituiert ist.

6. Verbindung nach einem der Ansprüche 1 bis 5, worin das optionale Anion ein Anion ist, das von einer aus der aus Chlorwasserstoff-, Bromwasserstoff-, Iodwasserstoff-, Schwefel-, schwefeliger, Salpeter-, salpetriger, Phosphor-, phosphoriger, Alkylsulfon- und Arylsulfonsäure bestehenden Gruppe ausgewählten Säure stammt.

7. Verbindung nach Anspruch 1, worin die Verbindung aus der Folgendes umfassenden Gruppe ausgewählt ist: 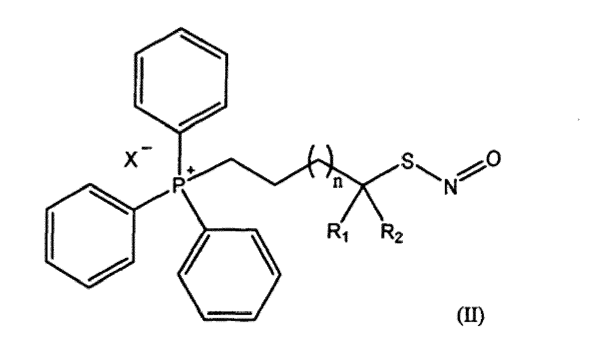 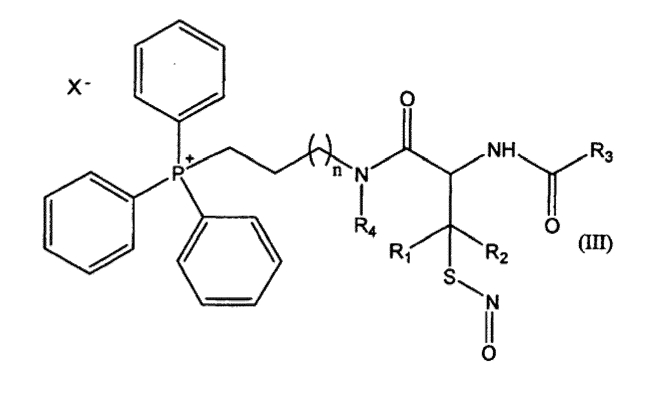 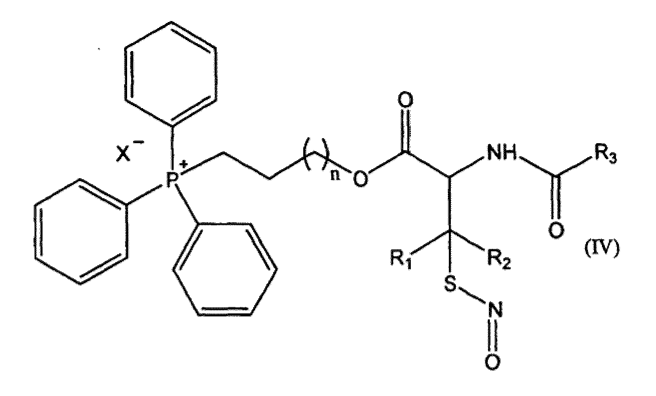 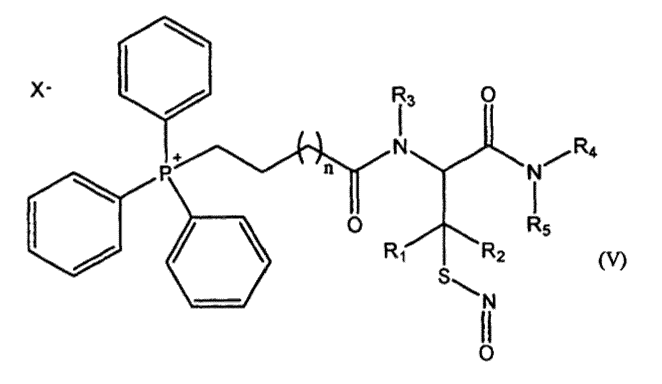 oder 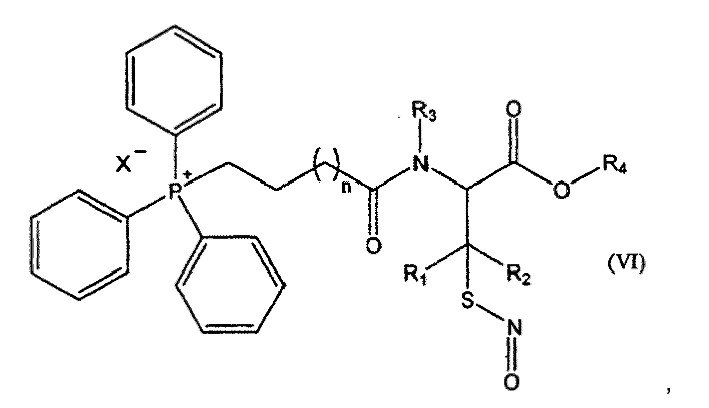 worin n = 0 bis 27 ist, X ein optionales Anion ist und R₁ und R₂ jeweils unabhängig voneinander aus der Wasserstoff, Alkyl und Aryl umfassenden Gruppe ausgewählt sind.

8. Verbindung nach Anspruch 1, ausgewählt aus der Folgendes umfassenden Gruppe:

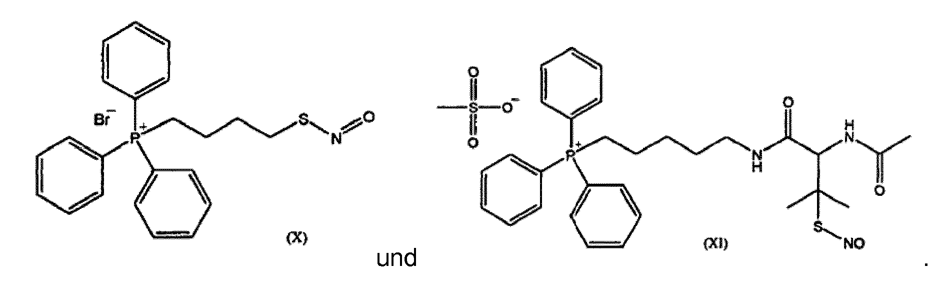

9. Pharmazeutische Zusammensetzung, umfassend eine therapeutisch wirksame Menge einer Verbindung nach einem der Ansprüche 1 bis 8 mit einem oder mehreren pharmazeutisch annehmbaren Exzipienten, Trägern oder Verdünnern.

10. Verwendung einer Verbindung nach einem der Ansprüche 1 bis 8 bei der Herstellung eines Medikaments zur Behandlung einer aus der aus Krebs, Neoplasmen, Tumorwachstum, Metastasen, Angina, Schlaganfall, Myokardinfarkt und Ischämie-Reperfusionsschaden bestehenden Gruppe ausgewählten Krankheit oder Störung.

11. Verwendung einer Verbindung nach einem der Ansprüche 1 bis 8 bei der Herstellung eines Medikaments zur Hemmung von Angiogenese bei einem Individuum.

12. Verwendung einer Verbindung nach einem der Ansprüche 1 bis 8 bei der Herstellung eines Medikaments zur Erzeugung von NO in den Mitochondrien eines Individuums.

13. Verwendung einer Verbindung nach einem der Ansprüche 1 bis 8 bei der Herstellung eines Medikaments zur Hemmung von Zytochromoxidase in den Mitochondrien eines Individuums.

14. Verwendung einer Verbindung nach einem der Ansprüche 1 bis 8 bei der Herstellung eines Medikaments zur S-Nitrosylierung von Proteinen in den Mitochondrien eines Individuums.

## Revendications

1. Composé de formule (I) 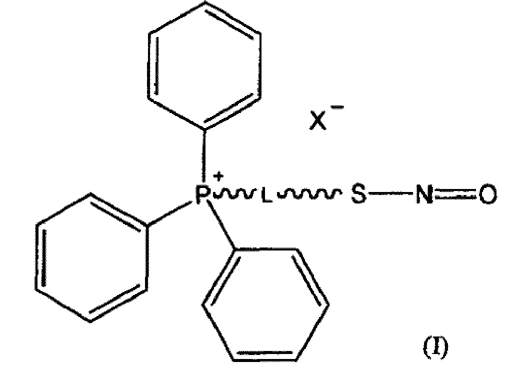 dans laquelle -L- est un groupe lieur et X est un anion facultatif; et
dans laquelle le groupe lieur est choisi dans l'ensemble comprenant
(a) un alkylène en C₁ à C₃₀
(b) un (alkylène en C₁ à Cₓ)-NR-(alkylène en C₁ à C_{y}), où R est H ou un alkyle ou aryle,
(c) un (alkylène en C₁ à Cₓ)-NR-C(=O)-(alkylène en C₁ à C_{y}), où R est H ou un alkyle ou aryle,
(d) un (alkylène en C₁ à Cₓ)-C(=O)-NR-(alkylène en C₁ à C_{y}), où R est H ou un alkyle ou aryle,
(e) un (alkylène en C₁ à Cₓ)-O-(alkylène en C₁ à C_{y}),
(f) un (alkylène en C₁ à Cₓ)-O-C(=O)-(alkylène en C₁ à C_{y}),
(g) un (alkylène en C₁ à Cₓ)-S-(alkylène en C₁ à C_{y}), et
(h) un (alkylène en C₁ à Cₓ)-aryl-(alkylène en C₁ à C_{y}),
où x + y = 30 et où l'alkylène est éventuellement substitué par un ou plusieurs groupes fonctionnels indépendamment choisis dans l'ensemble constitué par l'hydrogène, alkyle, cycloalkyle, alcényle, alcynyle, halogénoalkyle, aryle, aminoalkyle, hydroxyalkyle, alcoxyalkyle, alkylthio, alkylsulfinyle, alkylsulfonyle, carboxyalkyle, cyano, oxy, amino, alkylamino, amino-carbonyle, alcoxycarbonyle, aryloxycarbonyle, alkylamino-carbonyle, arylaminocarbonyle, aralkylaminocarbonyle, alkylcarbonylamino, arylcarbonylamino, aralkylcarbonyl-amino, alkylcarbonyle, hétérocyclocarbonyle, amino-sulfonyle, alkylaminosulfonyle, alkylsulfonyle et hétérocyclosulfonyle, ou bien les groupes substituants d'atomes de carbone adjacents dans le groupe lieur peuvent être pris conjointement avec les atomes de carbone auxquels ils sont rattachés pour former un carbocycle ou un hétérocycle.

2. Composé selon la revendication 1, dans lequel le groupe lieur est choisi dans l'ensemble comprenant
(a) un alkylène en C₁ à C₃₀,
(b) un (alkylène en C₁ à Cₓ)-NR-C(=O)-(alkylène en C₁ à C_{y}), où R est H ou un alkyle ou aryle,
(c) un (alkylène en C₁ à Cₓ)-C(=O)-NR-(alkylène en C₁ à C_{y}), où R est H ou un alkyle ou aryle, et
(d) un (alkylène en C₁ à Cₓ)-O-C(=O)-(alkylène en C₁ à C_{y}), où x + y = 30 et où l'alkylène est éventuellement substitué par un ou plusieurs groupes fonctionnels indépendamment choisis dans l'ensemble constitué par alkyle, cycloalkyle, alcényle, alcynyle, halogénoalkyle, aryle, aminoalkyle, hydroxyalkyle, alcoxyalkyle, alkylthio, alkylsulfinyle, alkylsulfonyle, carboxyalkyle, cyano, oxy, amino, alkylamino, aminocarbonyle, alcoxy-carbonyle, aryloxycarbonyle, alkylaminocarbonyle, aryl-aminocarbonyle, aralkylaminocarbonyle, alkylcarbonylamino, arylcarbonylamino, aralkylcarbonylamino, alkylcarbonyle, hétérocyclocarbonyle, aminosulfonyle, alkylaminosulfonyle, alkylsulfonyle et hétérocyclosulfonyle, ou bien les groupes substituants d'atomes de carbone adjacents dans le groupe lieur peuvent être pris conjointement avec les atomes de carbone auxquels ils sont rattachés pour former un carbocycle ou un hétérocycle.

3. Composé selon la revendication 2, dans lequel le groupe lieur est choisi dans l'ensemble comprenant
(a) un alkylène en C₁ à C₁₂,
(b) un (alkylène en C₁ à C₇)-NR-C(=O)-(alkylène en C₁ à C₅), où R est H ou un alkyle ou aryle,
(c) un (alkylène en C₁ à C₇)-C(=O)-NR-(alkylène en C₁ à C₅), où R est H ou un alkyle ou aryle, et
(d) un (alkylène en C₁ à C₇)-O-C(=O)-(alkylène en C₁ à C₅), où l'alkylène est éventuellement substitué par un ou plusieurs groupes fonctionnels indépendamment choisis dans l'ensemble constitué par alkyle, aryle, alcoxycarbonyle, aryloxycarbonyle, alkylaminocarbonyle, arylaminocarbonyle, alkylcarbonylamino, et arylcarbonylamino.

4. Composé selon l'une quelconque des revendications 1 à 3, dans lequel l'alkylène est substitué au niveau du carbone en β par rapport à l'atome de soufre par un ou plusieurs groupes fonctionnels indépendamment choisis dans l'ensemble constitué par alcoxycarbonyle, alkylaminocarbonyle et alkylcarbonylamino.

5. Composé selon l'une quelconque des revendications 1 à 4, dans lequel l'alkylène est substitué au niveau du carbone en α par rapport à l'atome de soufre par un alkyle ou aryle.

6. Composé selon l'une quelconque des revendications 1 à 5, dans lequel l'anion facultatif est un anion dérivé d'un acide choisi dans l'ensemble comprenant les acides chlorhydrique, bromhydrique, iodhydrique, sulfurique, sulfureux, nitrique, nitreux, phosphorique, phosphoreux, alkylsulfonique et arylsulfonique.

7. Composé selon la revendication 1, dans lequel le composé est choisi dans l'ensemble comprenant 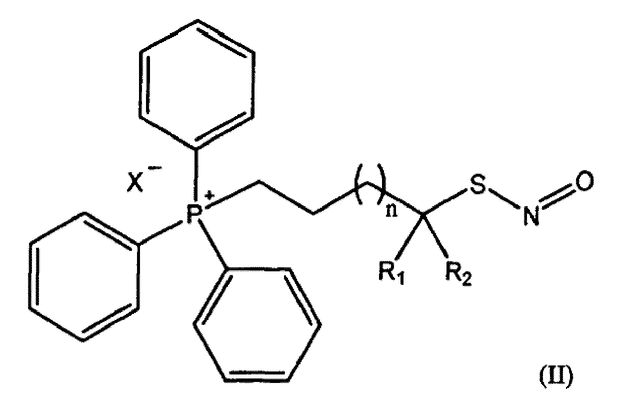 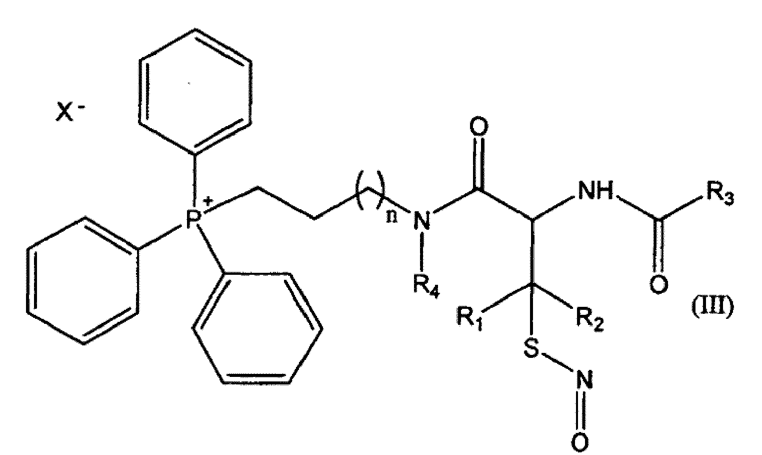 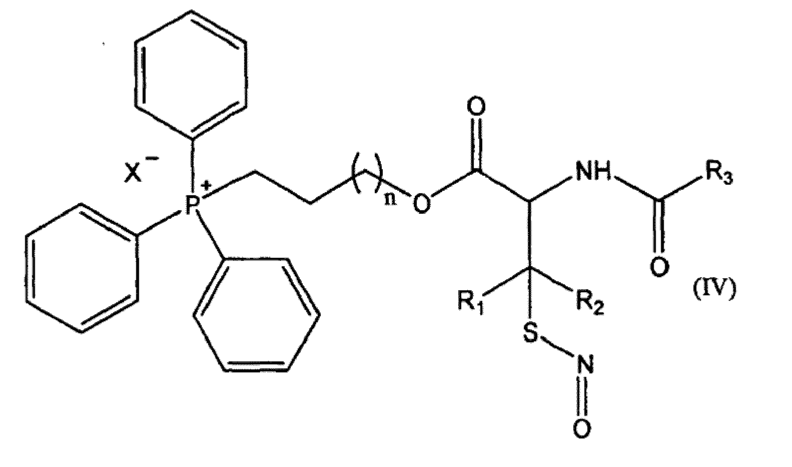 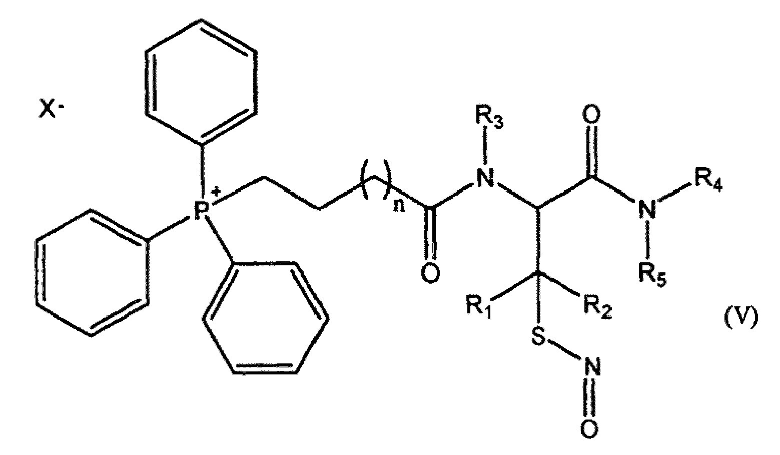 ou 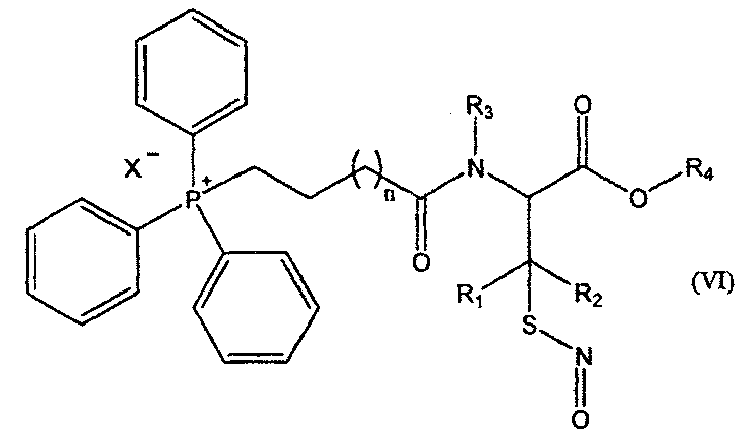 où n vaut de 0 à 27, X est un anion facultatif et R₁ et R₂ sont indépendamment choisis dans l'ensemble comprenant l'hydrogène, alkyle et aryle.

8. Composé selon la revendication 1, choisi dans le groupe comprenant 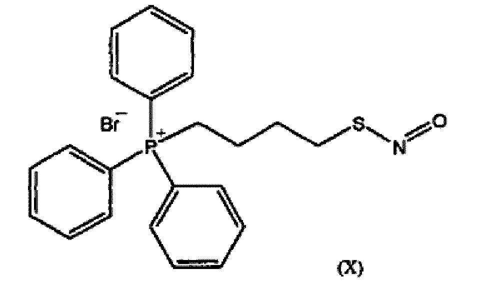 et

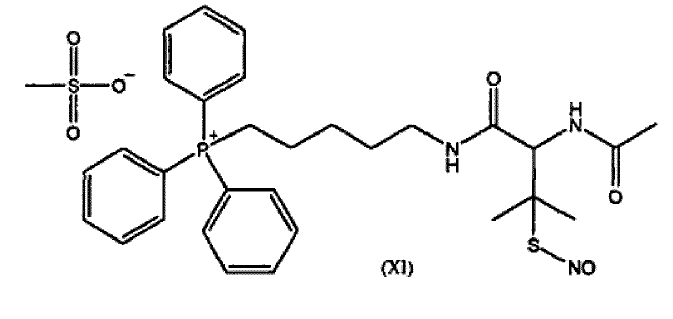

9. Composition pharmaceutique comprenant une quantité efficace, du point de vue thérapeutique, d'un composé selon l'une quelconque des revendications 1 à 8, avec un ou plusieurs excipients, véhicules ou diluants pharmaceutiquement acceptables.

10. Utilisation d'un composé de l'une quelconque des revendications 1 à 8 dans la préparation d'un médicament pour le traitement d'une maladie ou d'un trouble choisi dans le groupe comprenant un cancer, les néoplasmes, la croissance d'une tumeur, une métastase, une angine, un accident cérébro-vasculaire, un infarctus du myocarde et une lésion ischémique par reperfusion.

11. Utilisation d'un composé de l'une quelconque des revendications 1 à 8 dans la préparation d'un médicament pour inhiber l'angiogenèse chez un sujet.

12. Utilisation d'un composé de l'une quelconque des revendications 1 à 8 dans la préparation d'un médicament pour générer du NO dans les mitochondries d'un sujet.

13. Utilisation d'un composé de l'une quelconque des revendications 1 à 8 dans la préparation d'un médicament pour inhiber la cytochrome oxydase dans les mitochondries d'un sujet.

14. Utilisation d'un composé de l'une quelconque des revendications 1 à 8 dans la préparation d'un médicament pour S-nitrosyler des protéines dans les mitochondries d'un sujet.
